# EUROPÄISCHE PATENTSCHRIFT

(11) **EP 0 672 347 B1**
(45) Veröffentlichungstag und Bekanntmachung des Hinweises auf die Patenterteilung: **28.10.1998**
(21) Anmeldenummer: 95108494.6
(22) Anmeldetag: 09.07.1993
(51) Int. Cl.: A01N 37/38, A01N 37/50

(54) **Substituierte Oximether und ihre Verwendung zur Bekämpfung von Schädlingen**
Substituted oxime-ethers and their use as pesticides
Ethers d'oximes substitués et leur application comme pesticides

(30) Priorität: 15.07.1992 DE 4223210; 30.09.1992 DE 4232816; 31.03.1993 DE 4310495
(43) Veröffentlichungstag der Anmeldung: 20.09.1995
(62) Teilanmeldung aus: 93110979.7
(73) Patentinhaber: BASF AKTIENGESELLSCHAFT, 67056 Ludwigshafen (DE)
(72) Erfinder: Oberdorf, Klaus, Dr., D-6900 Heidelberg (DE); Sauter, Hubert, Dr., D-6800 Mannheim 1 (DE); Grammenos, Wassilios, Dr., D-67063 Ludwigshafen (DE); Kirstgen, Reinhard, Dr., D-6730 Neustadt (DE); Harries, Volker, Dr., D-6710 Frankenthal (DE); Lorenz, Gisela, Dr., D-6730 Neustadt (DE); Ammermann, Eberhard, Dr., D-6148 Heppenheim (DE); Gold, Randall Evan, Dr., D-67117 Limburgerhof (DE); Siegel, Wolfgang, Dr., D-6800 Mannheim (DE); Harreus, Albrecht, Dr., D-6700 Ludwigshafen (DE)

(56) Entgegenhaltungen:
- EP-A- 0 335 519
- EP-A- 0 386 561

## Beschreibung

Die vorliegende Erfindung betrifft die Verwendung von Verbindungen der allgemeinen Formel IA, 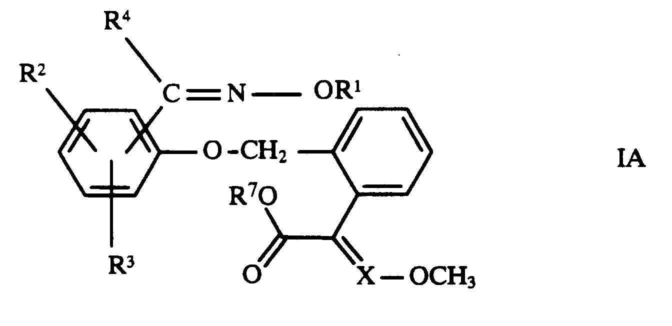 in der
R¹
   C₁-C₆-Alkyl, C₃-C₆-Alkenyl, C₃-C₄-Alkinyl, C₁-C₆-Halogenalkyl, C₃-C₆-Halogenalkenyl, C₁-C₄-Alkoxy-C₁-C₆-alkyl, C₃-C₆-Cycloalkyl, C₃-C₆-Cycloalkyl-C₁-C₄-alkyl, Cyan-C₁-C₆-alkyl, C₁-C₆-Alkoxycarbonyl-C₁-C₆-alkyl, Aryl-C₁-C₆-alkyl, Heteroaryl-C₁-C₆-alkyl, Aryl-C₃-C₆-alkenyl oder Aryloxy-C₁-C₆-alkyl bedeutet, wobei der aromatische oder heteroaromatische Ring gegebenenfalls durch einen oder mehrere der folgenden Reste substituiert ist:
   C₁-C₄-Alkyl, C₁-C₂-Halogenalkyl, C₃-C₆-Cycloalkyl, C₁-C₄-Alkoxy, C₁-C₂-Halogenalkoxy, Halogen, Aryl, Aryloxy,
R² und R³
   gleich oder verschiedenen sind und Wasserstoff, C₁-C₄-Alkyl, C₁-C₂-Halogenalkyl, C₁-C₄-Alkoxy, C₁-C₂-Halogenalkoxy, Halogen, Cyano oder Nitro bedeuten,
R⁴
   Wasserstoff, C₁-C₆-Alkyl, C₁-C₆-Cycloalkyl, C₁-C₇-Halogenalkyl oder Aryl bedeutet, wobei der aromatische Ring gegebenenfalls durch einen oder mehrere der folgenden Reste substituiert ist: C₁-C₄-Alkyl, C₁-C₂-Halogenalkyl, C₁-C₄-Alkoxy, C₁-C₂-Halogenalkoxy, Halogen, Cyano oder Nitro,
R⁷ C₁-C₄-Alkyl bedeutet, und
X CH oder N bedeutet,
zur Bekämpfung von Schädlingen aus der Klasse der Insekten, Spinnentiere und Nematoden.

Es ist bekannt, Oximether wie zum Beispiel das 2-(2'-Methylphenoxymethyl)-phenyl-glyoxylsäuremethylester-O-methyloxim oder das 2-(2'-Methyl-4'-(methoximinoeth-1''-yl)-phenoxymethyl)-phenylglyoxylsäuremethylester-O-methyloxim als Fungizide zu verwenden (EP-A 253 213; EP-A 398 692).

Des weiteren sind aus der EP-A 386 561 Verbindungen der Formel IA als fungizide Wirkstoffe bekannt.

Aufgabe der vorliegenden Erfindung waren neue Verbindungen mit breiterer Anwendbarkeit im Pflanzenschutz.

Demgemäß wurden Verfahren zur Bekämpfung der vorstehend genannten Schädlinge und die Verwendung der eingangs definierten Verbindungen der Formel IA zur Bekämpfung der Schädlinge gefunden.

Die in der allgemeinen Formel IA aufgeführten Reste können beispielsweise folgende Bedeutung haben:
R¹
   kann z.B. C₁-C₆-Alkyl (C₁-C₄-Alkyl) (z.B. Methyl, Ethyl, n- oder iso-Propyl, n-, iso-, sec.- oder tert. -Butyl, n-, iso-, sec.-, tert.-oder neo-Pentyl, Hexyl), C₃-C₆-Alkenyl (z.B. Allyl, 2-Butenyl, 3-Butenyl, 1-Methyl-2-propenyl, 2-Methyl-2-propenyl), C₃-C₄-Alkinyl (z.B. Propargyl, 2-Butinyl), C₁-C₆-Halogenalkyl, (z.B. 2-Fluorethyl), C₃-C₆-Halogenalkenyl (z.B. 3-Chlorallyl), C₁-C₄-Alkoxy-C₁-C₆-alkyl (z.B. 2-Methoxyethyl, 3-Ethoxypropyl), C₃-C₆-Cycloalkyl (z.B. Cyclopropyl, Cyclobutyl, Cyclopentyl, Cyclohexyl), C₃-C₆-Cycloalkyl-C₁-C₄-alkyl (z.B. Cyclopropylmethyl, Cyclohexylmethyl), Cyan-C₁-C₆-alkyl (z.B. Cyanmethyl, 3-Cyanpropyl), C₁-C₆-Alkoxycarbonyl-C₁-C₆-alkyl (z.B. Ethoxycarbonylmethyl, tert.-Butoxycarbonylmethyl, tert.-Butoxycarbonylpropyl), Aryl(Phenyl)-C₁-C₆-alkyl (z.B. Benzyl, 2-Phenylethyl, 3-Phenylpropyl, 4-Phenylbutyl), Heteroaryl-(Pyridyl, Thienyl)-C₁-C₆-alkyl (z.B. Pyrid-3-yl-methyl, Thien-2-yl-methyl), Aryl-(Phenyl)-C₃-C₆-alkenyl (z.B. 4-Phenyl-2-butenyl, 4-Phenyl-3-butenyl), Aryloxy(Phenoxy)-C₁-C₆-alkyl (z.B. Phenoxymethyl, Phenoxyethyl, Phenoxypropyl, Phenoxybutyl, Naphthoxymethyl, Naphthoxyethyl) sein,
   wobei der aromatische (Phenyl) oder heteroaromatische (Pyridyl, Thienyl) Ring gegebenenfalls durch einen oder mehrere z.B. 1 bis 5, insbesondere 1 bis 3 der folgenden Reste substituiert ist:
   C₁-C₄-Alkyl (z.B. Methyl, Ethyl, Propyl, Butyl), C₁-C₂-Halogenalkyl, (z.B. Trifluormethyl, Trichlormethyl), C₃-C₆-Cycloalkyl (z.B. Cyclopropyl, Cyclobutyl, Cyclopentyl, Cyclohexyl), C₁-C₄-Alkoxy (z. B. Methoxy, Ethoxy, Propoxy, Butoxy), C₁-C₂-Halogenalkoxy (z. B. Trifluormethoxy), Halogen (z.B. Fluor, Chlor, Brom), Aryl (z.B. Phenyl), Arylory (z.B. Phenoxy),
R² und R³
   können gleich oder verschieden sein und Wasserstoff, C₁-C₄-Alkyl (z.B. Methyl, Ethyl, n- oder iso-Propyl, Butyl), C₁-C₂-Halogenalkyl, (z.B. Trifluormethyl, Trichlormethyl), C₁-C₄-Alkoxy (z.B. Methoxy, Ethoxy, n-oder iso-Propoxy, Butoxy), C₁-C₂-Halogenalkoxy (z. B. Trifluormethoxy), Halogen (z.B. Fluor, Chlor, Brom, Jod), Cyano oder Nitro sein,
R⁴
   kann z. B. C₁-C₆-Alkyl, (C₁-C₄-Alkyl) (z.B. Methyl, Ethyl, n- oder iso-Propyl, n-, iso-, sec.- oder tert.-Butyl, n-, iso, sec.-tert. oder neo-Pentyl, Hexyl), C₁-C₇-Halogenalkyl (z.B. Trifluormethyl, Trichlormethyl, Chlormethyl, 2-Chlorethyl, 3-Chlorpropyl, 3-Brompropyl, 4-Chlorbutyl, 4-Brombutyl, 5-Chlorpentyl, 5-Brompentyl, 6-Chlorhexyl, 6-Bromhexyl), C₃-C₆-Cycloalkyl (z.B. Cyclopropyl, Cyclobutyl, Cyclopentyl und Cyclohexyl) sein oder Aryl (z.B. Phenyl) sein, wobei der aromatische Ring gegebenenfalls durch einen oder mehrere z.B. 1 bis 5, insbesondere 1 bis 3 der folgenden Reste substituiert ist: C₁-C₄-Alkyl (z.B. Methyl, Ethyl, Propyl, Butyl), C₁-C₂-Halogenalkyl (z.B. Trifluormethyl, Trichlormethyl), C₁-C₄-Alkoxy (z.B. Methoxy, Ethoxy, Propoxy, Butoxy), C₁-C₂-Halogenalkoxy (z.B. Difluormethoxy, Trifluormethoxy), Halogen (z.B. Fluor, Chlor, Brom, Jod) Cyano oder Nitro.
X kann CH oder N bedeuten und
R⁷ kann C₁-C₄-Alkyl wie vorstehend genannt, insbesondere Methyl bedeuten.

Der Rest -C(R⁴)=N-O-R¹ kann am Phenylrest im Hinblick auf den Rest -O-CH₂- in 2-, oder in 3- oder bevorzugt in 4-Stellung stehen.

Die Verbindungen der allgemeinen Formel IA können bei der Herstellung aufgrund der C=C- bzw. C=N-Doppelbindungen als E/Z-Isomerengemische anfallen. Diese können in der üblichen Weise, z.B. durch Kristallisation oder Chromatographie, in die einzelnen Komponenten getrennt werden. Sowohl die einzelnen isomeren Verbindungen als auch ihre Gemische werden von der Erfindung umfaßt und sind als Schädlingsbekämpfungsmittel brauchbar. Bezüglich der Gruppierung -C(R⁴)=N-OR¹ sind diejenigen Verbindungen bevorzugt, in denen R⁴ und OR¹ an der C=N-Doppelbindung cis-ständig sind und in denen deshalb bei kleinen Substituenten wie z.B. Methyl die C=N-Doppelbindung E-Konfiguration hat.

Die Verbindungen der Formel IA sind aus der EP-A 386 561 bekannt oder lassen sich nach den dort beschriebenen Methoden herstellen.

Die folgenden Beispiele und Vorschriften sollen die Herstellung der neuen Wirkstoffe und ihrer Vorprodukte erläutern.

### Herstellungsbeispiel 1

### 2-[2'-Methyl-4'-(methoxyiminoeth-1''-yl)-phenoxymethyl]-phenylglyoxylsäure-methylester-O-methyloxim

a) 225,3 g (1,5 mol) 4-Hydroxy-3-methyl-acetophenon werden in 600 ml trockenem Methanol gelöst. 150,3 g (1,8 mol) Methoxyaminhydrochlorid und 100 g Molekularsieb werden zugesetzt. Es wird 12 Stunden bei Raumtemperatur (20°C) gerührt. Das Molekularsieb wird abfiltriert. Das Filtrat wird eingeengt. Der verbleibende Rückstand wird in Dichlormethan aufgenommen. Die organische Phase wird mit Wasser gewaschen, getrocknet und eingeengt. Das erhaltene Festprodukt wird mit Pentan gewaschen und anschließend getrocknet. Man erhält 252 g (94 %) 4-Hydroxy-2-methyl-acetophenon-O-methyloxim in Form eines farblosen kristallinen Feststoffs (Fp.: 96 - 98°C).
b) 89,6 g (0,5 mol) 4-Hydroxy-3-methyl-acetophenon-O-methyloxim werden unter Stickstoff in 300 ml trockenem Methanol vorgelegt. 90 g (0,5 mol) einer 30 % (Gew.-%) Natriummethanolat-Lösung werden zugetropft. Nach 2 Stunden wird das Methanol abdestilliert. Der Rückstand wird in 700 ml Dimethylformamid gelöst. 15 g Kaliumjodid werden zugesetzt. Anschließend wird bei Raumtemperatur unter Stickstoff eine Lösung von 151,6 g (0,53 mol) 2-(Brommethyl)-phenylglyoxylsäuremethylester-O-methyloxim in 300 ml Methanol zugetropft. Nach etwa 10 Stunden Rühren bei Raumtemperatur wird auf etwa 10 °C abgekühlt und es wird Wasser zugetropft. Der entstandene Niederschlag wird abfiltriert, mit Wasser und Pentan nachgewaschen und getrocknet. Man erhält 153,7 g (80 %) 2-[2'-Methyl-4'-(methoxyiminoeth-1''-yl)-phenoxymethyl]-phenylglyoxylsäuremethylester-O-methyloxim als farblosen kristallinen Feststoff (Fp.: 138 - 140°C).

### Herstellungsbeispiel 2

### α-[2-(2'-Methyl-4'-(methoxyiminoeth-1''-yl)-phenoxymethyl)phenyl]-β-methoxy-acrylsäure-methylester

α-(2-Brommethylphenyl)-β-methoxy-acrylsäuremethylester und 4-Hydroxy-3-methyl-acetophenon-O-methyloxim werden analog Vorschrift b) (Beispiel 1) zu α-[2-(2'-Methyl-4'-(methoxyiminoeth-1''-yl)-phenoxymethyl)-phenyl] -β-methoxy-acrylsäure-methylester umgesetzt. Die Verbindung fällt als farbloser Feststoff (Fp.: 118 - 120°C) an.

In entsprechender Weise lassen sich die in der folgenden Tabelle zusammengestellten Verbindungen IA herstellen. Die Verbindungen IA sind gemäß den Angaben der EP-A 386 561 erhältlich. Sie sind ebenfalls in der folgenden Tabelle aufgeführt.

Desweiteren sind in den anschließenden Tabellen 3, 4, 12-17 und 23-27 diejenigen Verbindungen IA zusammengestellt, denen im Hinblick auf ihre biologische Wirksamkeit gegen tierische Schädlinge (Insekten, Spinnentiere und Nematoden) eine besondere Bedeutung zukommt.

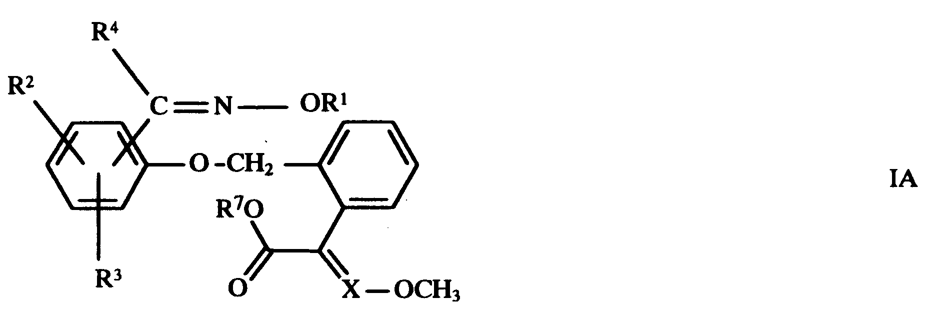

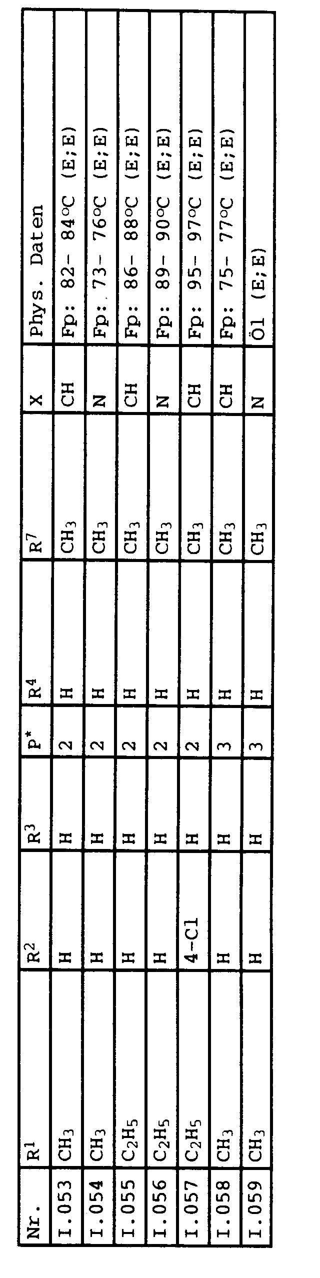

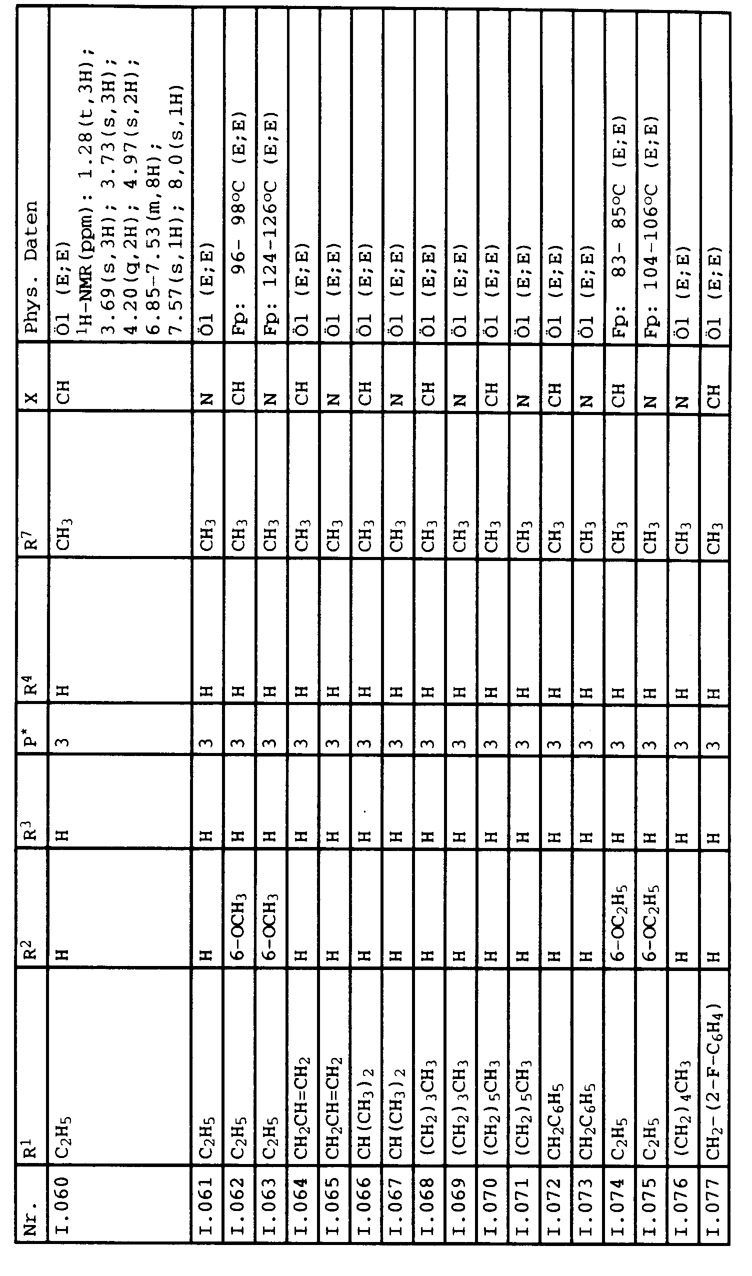

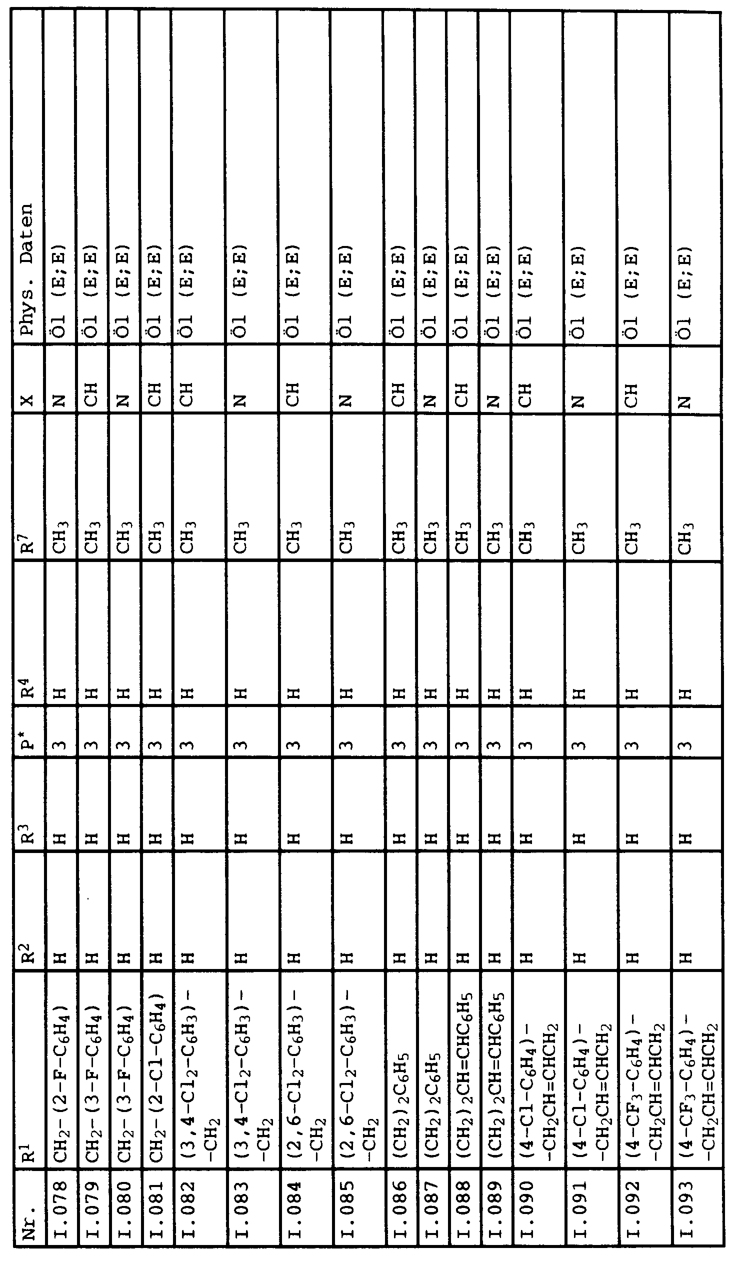

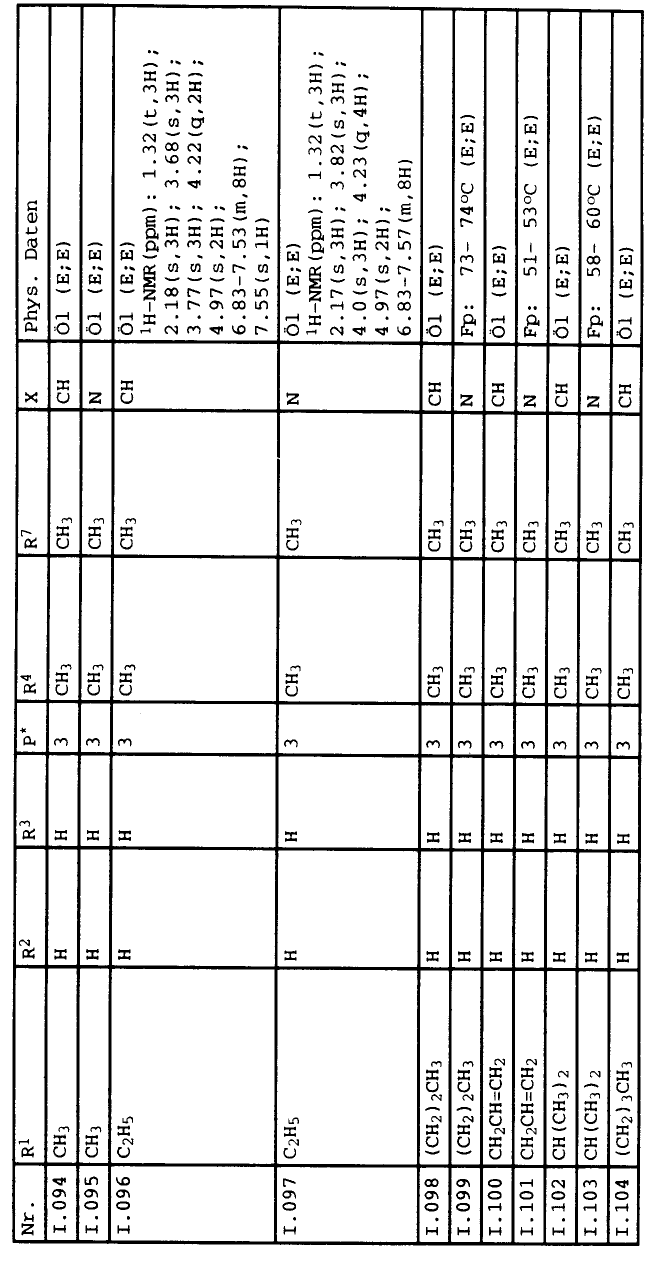

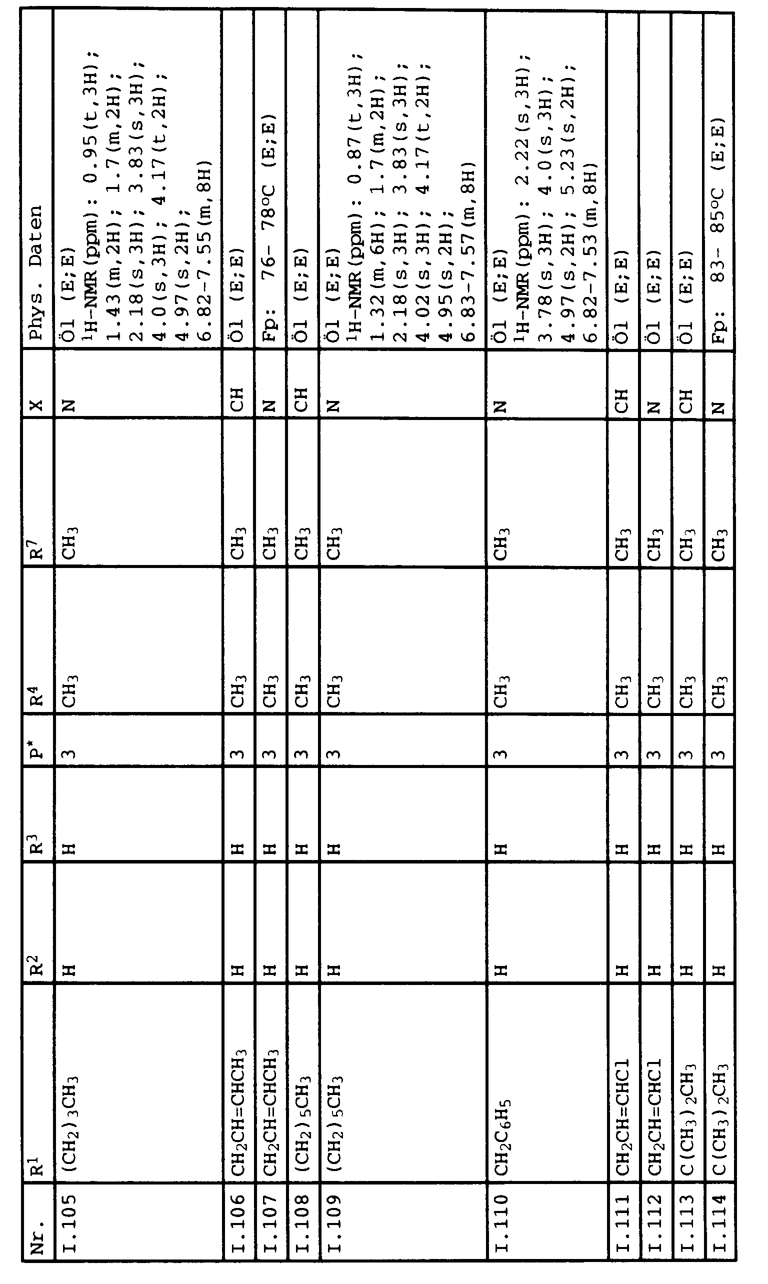

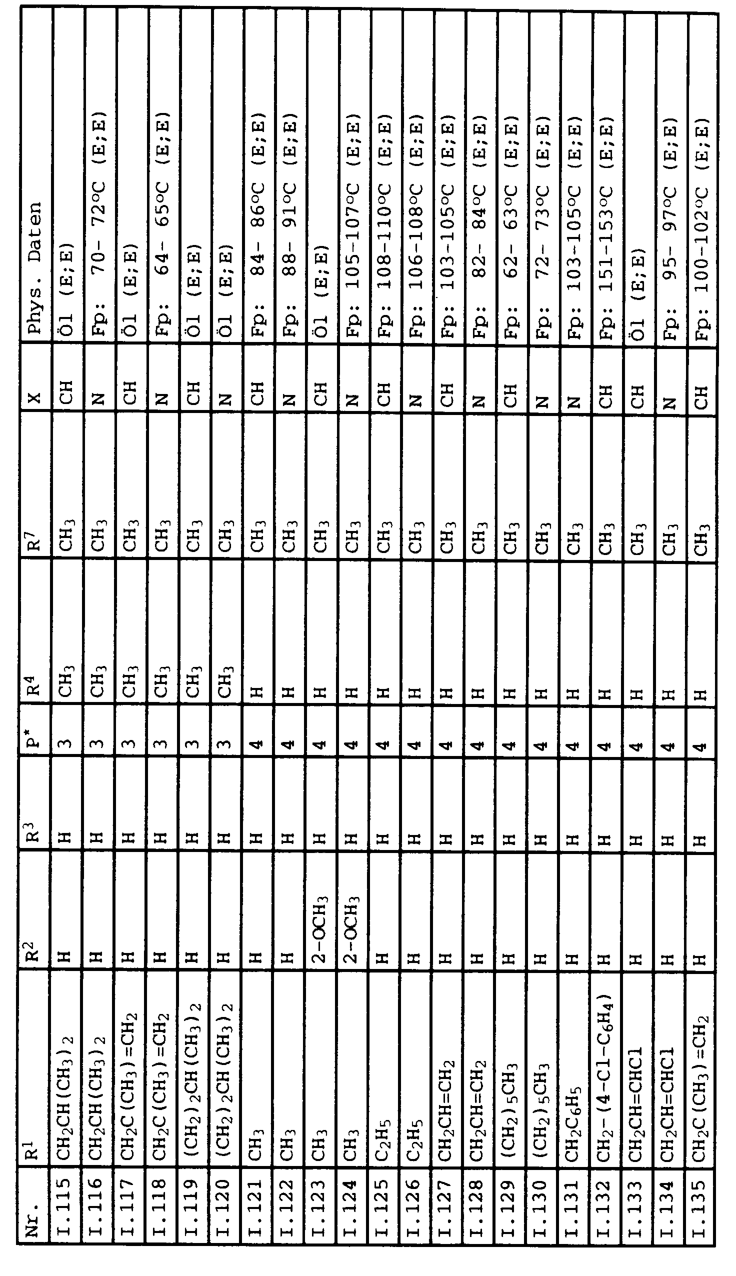

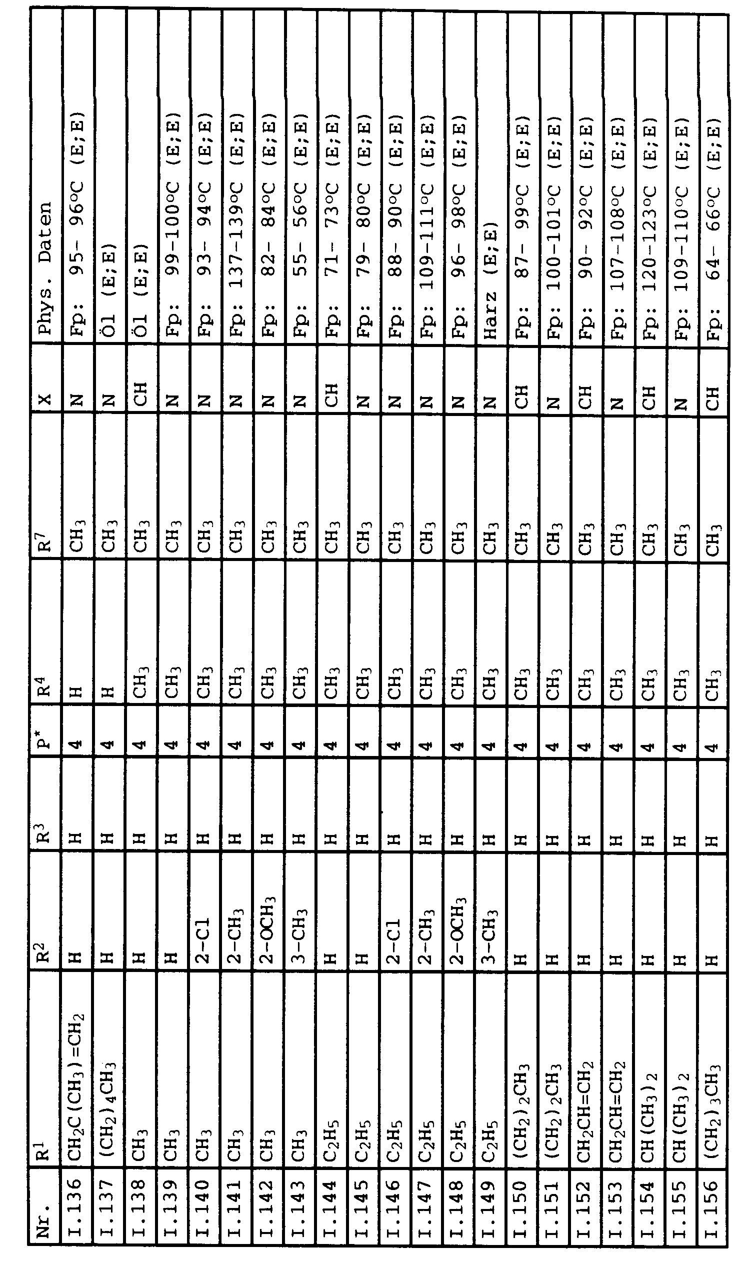

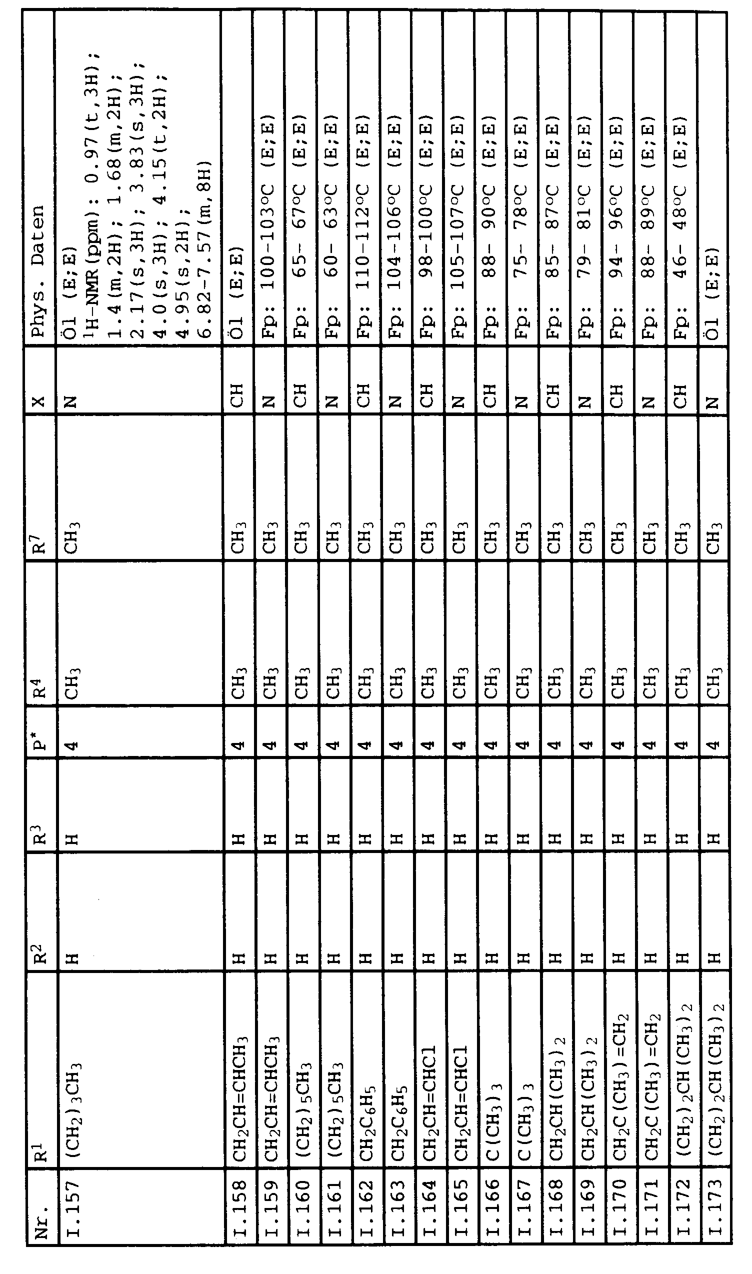

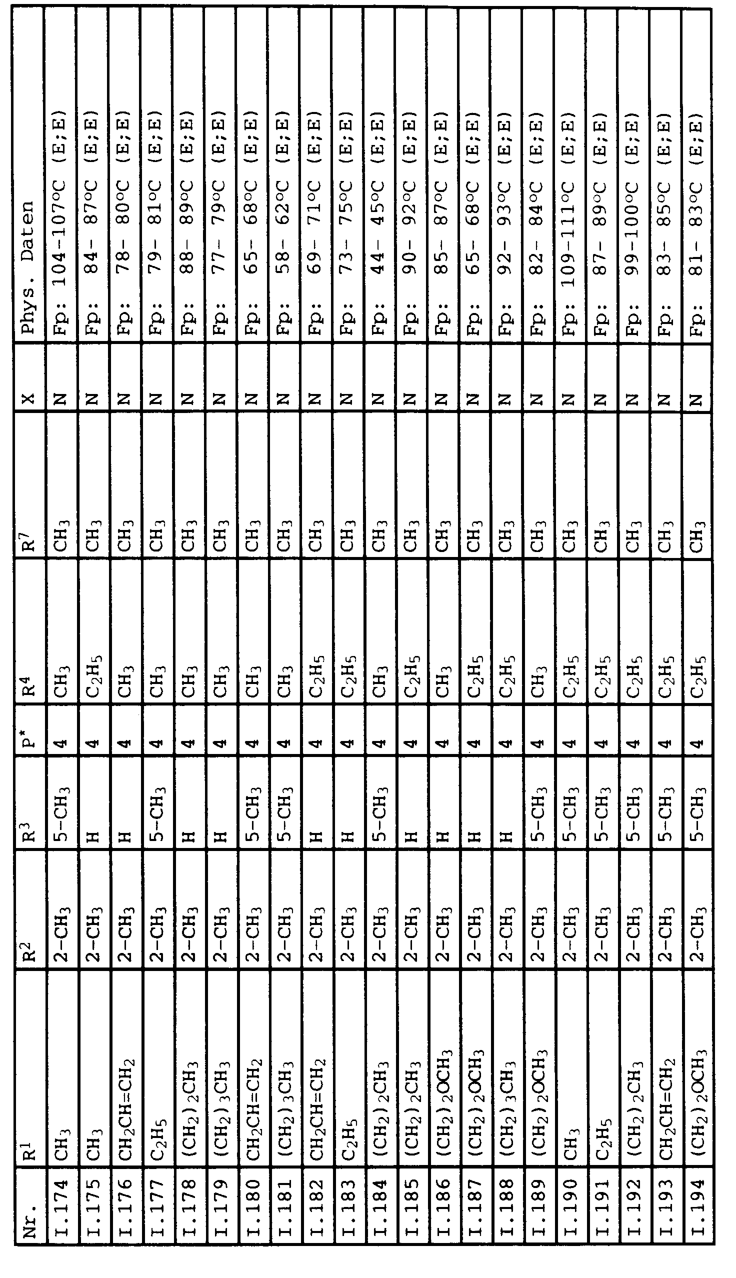

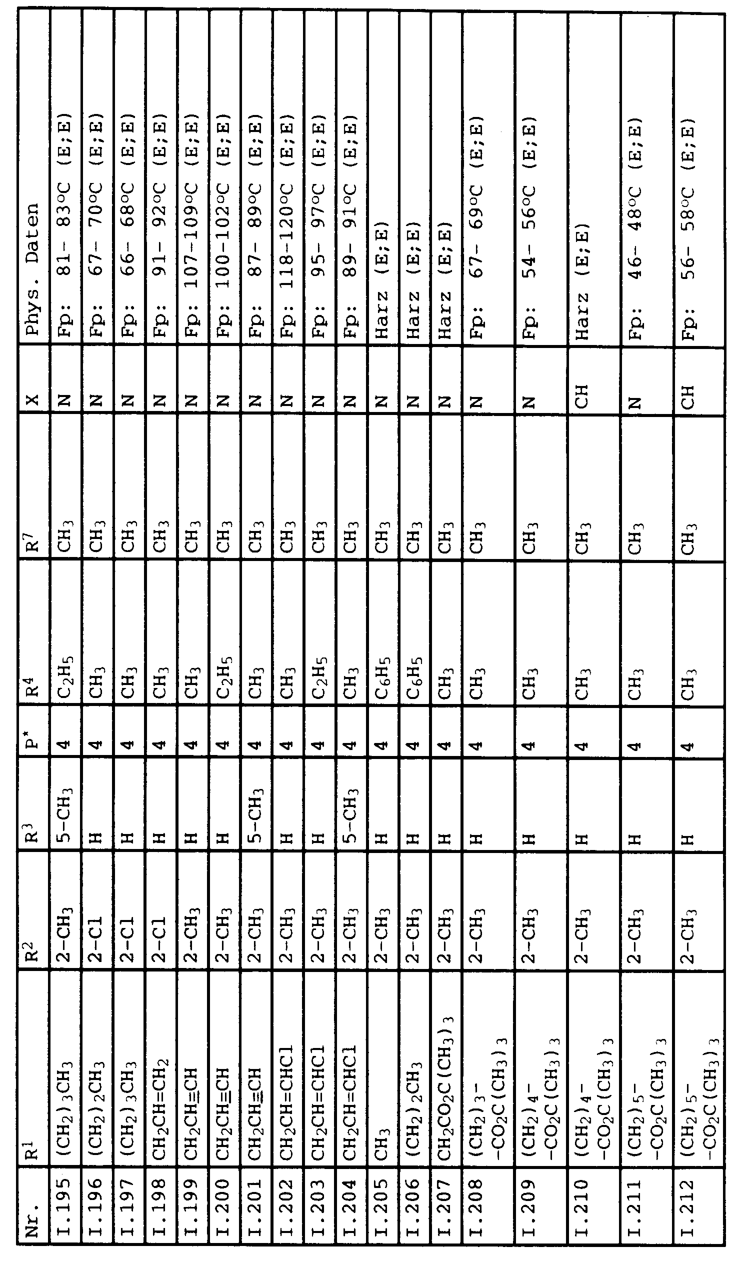

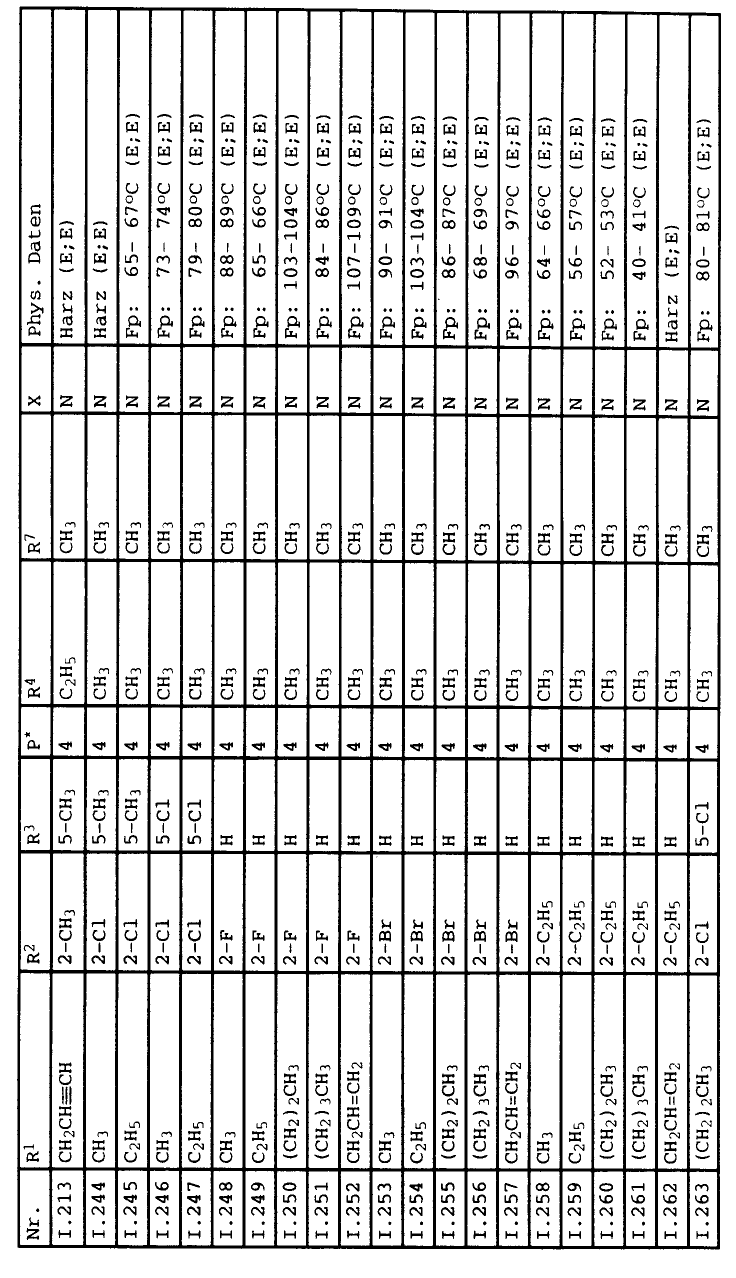

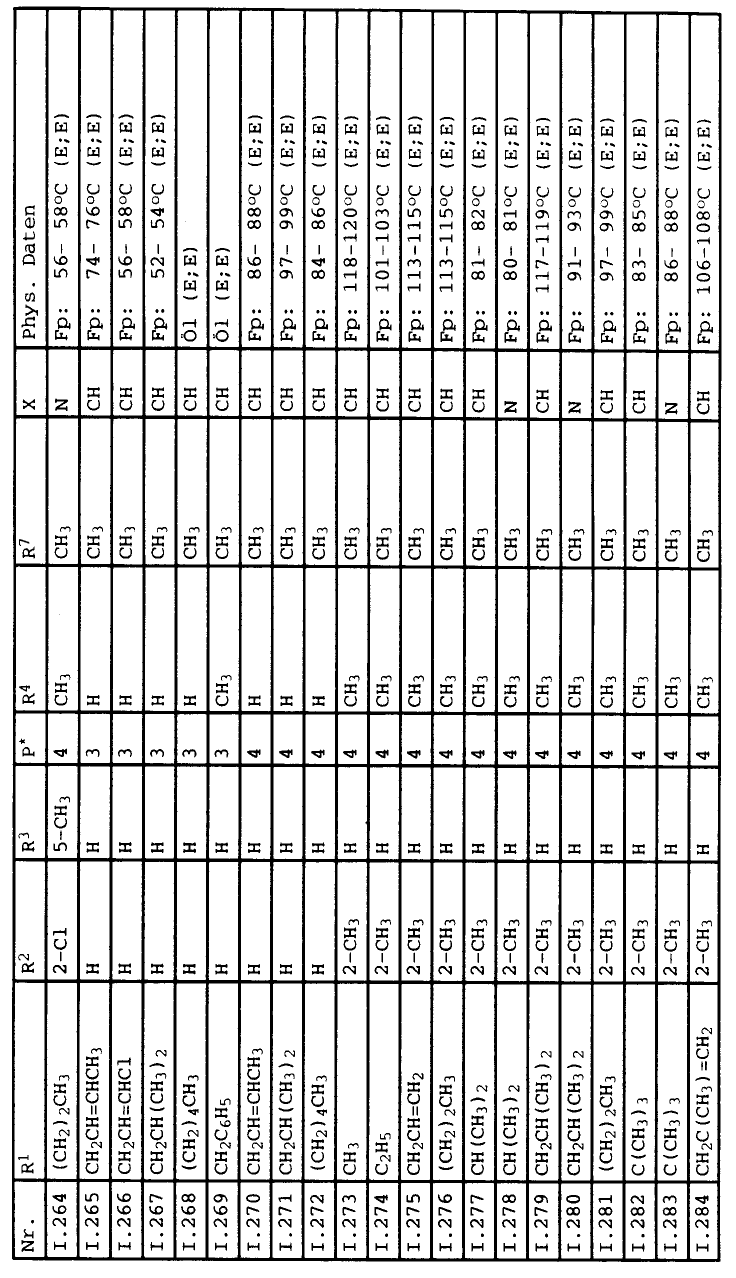

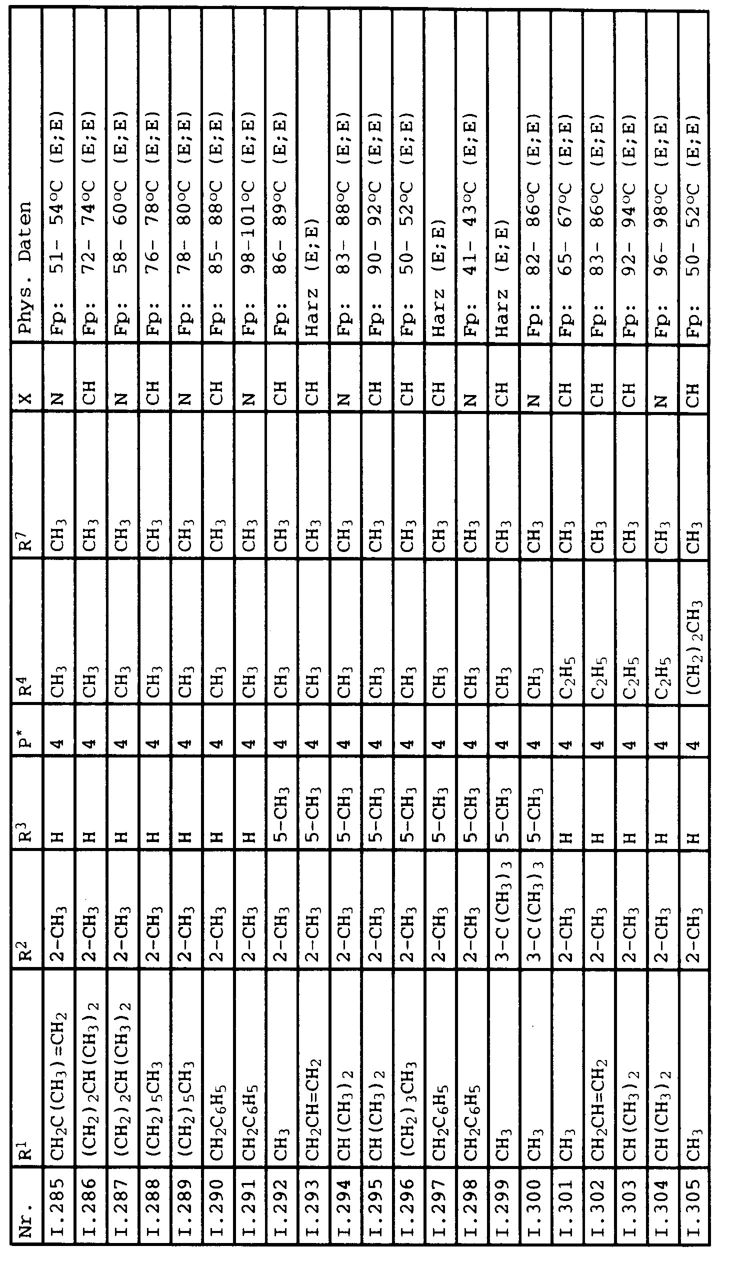

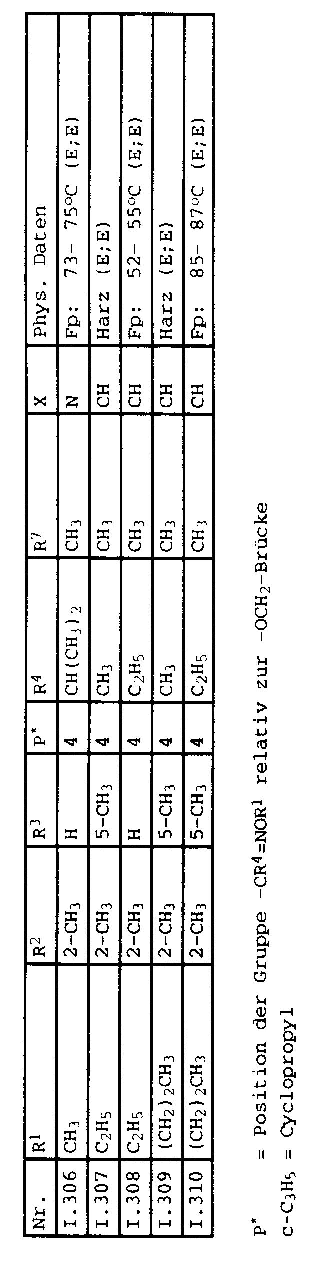

- Tabelle 3:: Verbindungen der allgemeinen Formel I.3, in denen die Kombination der Substituenten R¹, R², R³, R⁴ und X für eine Verbindung jeweils einer Zeile der Tabelle A entspricht 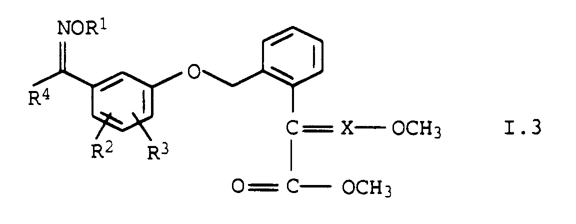
- Tabelle 4:: Verbindungen der allgemeinen Formel I.4, in denen die Kombination der Substituenten R¹, R², R³, R⁴ und X für eine Verbindung jeweils einer Zeile der Tabelle B entspricht 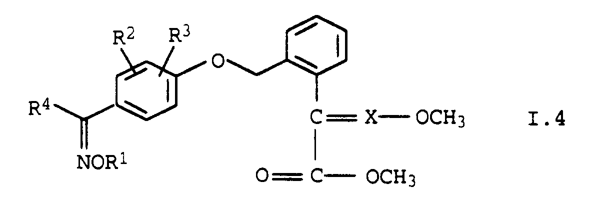
- Tabelle 12:: Verbindungen der allgemeinen Formel I.4, in denen R⁴ Cyclopropyl bedeutet und die Kombination der Substituenten R¹, R², R³ und X für eine Verbindung jeweils einer Zeile der Tabelle D entspricht
- Tabelle 13:: Verbindungen der allgemeinen Formel I.4, in denen R⁴ Cyclopentyl bedeutet und die Kombination der Substituenten R¹, R², R³ und X für eine Verbindung jeweils einer Zeile der Tabelle D entspricht
- Tabelle 14:: Verbindungen der allgemeinen Formel I.4, in denen R⁴ Cyclohexyl bedeutet und die Kombination der Substituenten R¹, R², R³ und X für eine Verbindung jeweils einer Zeile der Tabelle D entspricht
- Tabelle 15:: Verbindungen der allgemeinen Formel I.4, in denen R⁴ CF₃ bedeutet und die Kombination der Substituenten R¹, R², R³ und X für eine Verbindung jeweils einer Zeile der Tabelle D entspricht
- Tabelle 16:: Verbindungen der allgemeinen Formel I.4, in denen R⁴ CH₂Cl bedeutet und die Kombination der Substituenten R¹, R², R³ und X für eine Verbindung jeweils einer Zeile der Tabelle D entspricht
- Tabelle 17:: Verbindungen der allgemeinen Formel I.4, in denen R⁴ CH₂CH₂Cl bedeutet und die Kombination der Substituenten R¹, R², R³ und X für eine Verbindung jeweils einer Zeile der Tabelle D entspricht
- Tabelle 23:: Verbindungen der allgemeinen Formel I.4, in denen R⁴ für Cyclopropyl und =X- für =N- stehen und die Kombination der Substituenten R¹, R² und R³ für eine Verbindung jeweils einer Zeile der Tabelle E entspricht
- Tabelle 24:: Verbindungen der allgemeinen Formel I.4, in denen R⁴ für Cyclopentyl und =X- für =N- stehen und die Kombination der Substituenten R¹, R² und R³ für eine Verbindung jeweils einer Zeile der Tabelle E entspricht
- Tabelle 25:: Verbindungen der allgemeinen Formel I.4, in denen R⁴ für Cyclohexyl und =X- für =N- stehen und die Kombination der Substituenten R¹, R² und R³ für eine Verbindung jeweils einer Zeile der Tabelle E entspricht
- Tabelle 26:: Verbindungen der allgemeinen Formel I.4, in denen R⁴ für CF₃ und =X- für =N- stehen und die Kombination der Substituenten R¹, R² und R³ für eine Verbindung jeweils einer Zeile der Tabelle E entspricht
- Tabelle 27:: Verbindungen der allgemeinen Formel I.4, in denen R⁴ für CH₂CH₂Cl und =X- für =N- stehen und die Kombination der Substituenten R¹, R² und R³ für eine Verbindung jeweils einer Zeile der Tabelle E entspricht

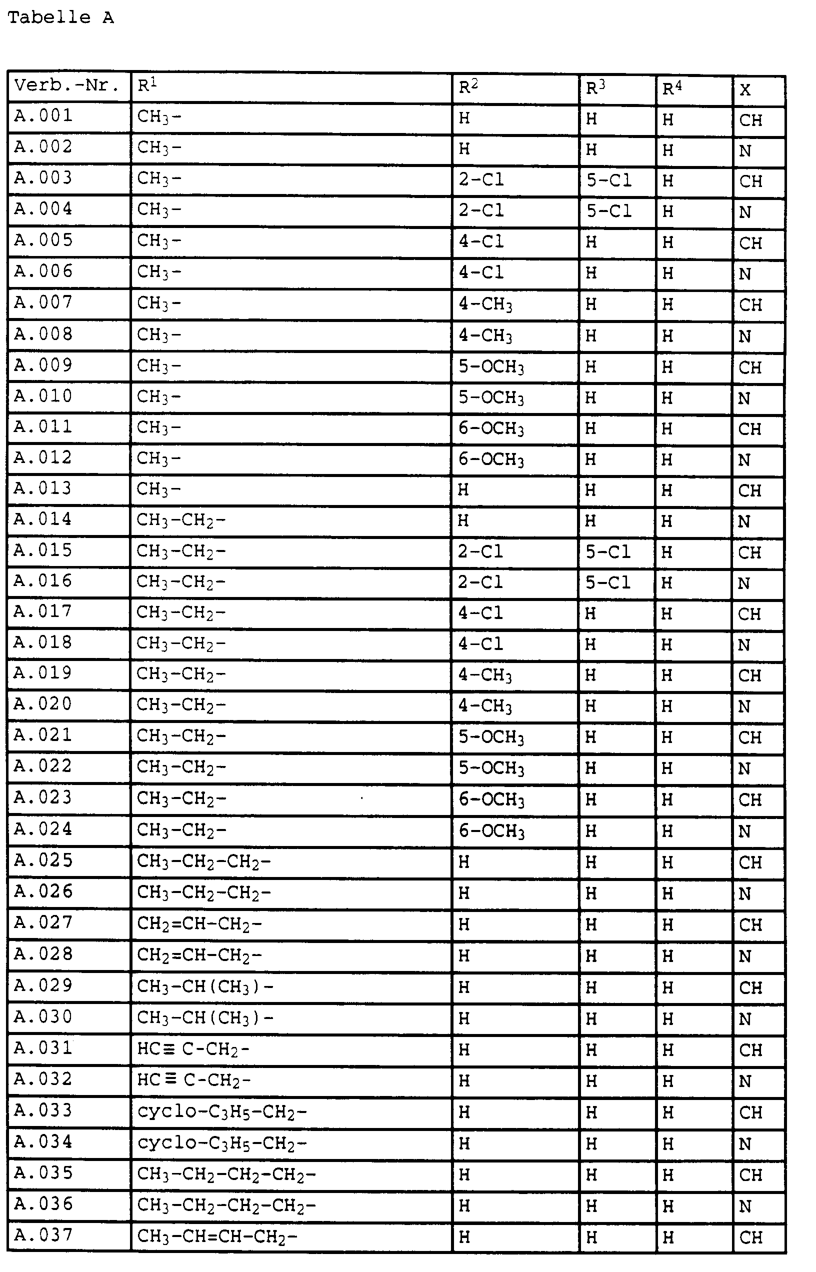

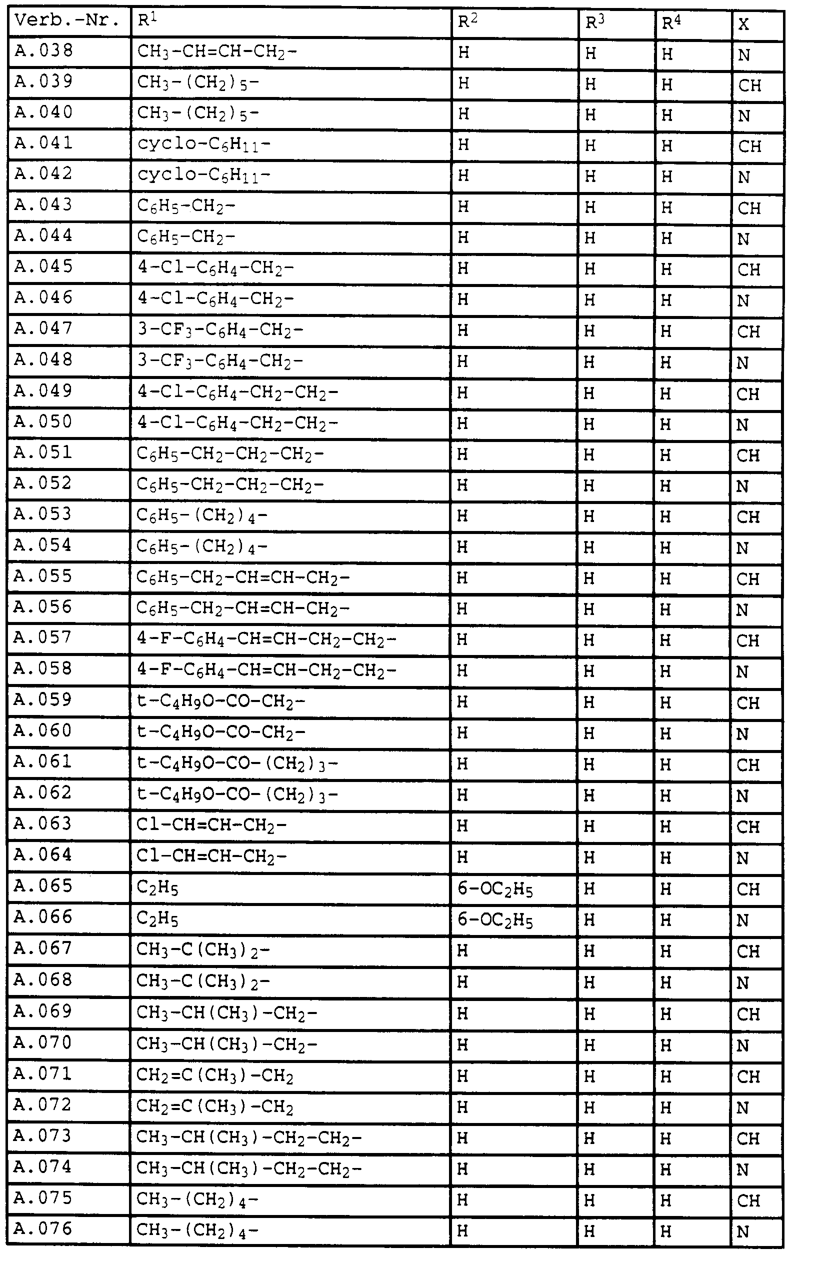

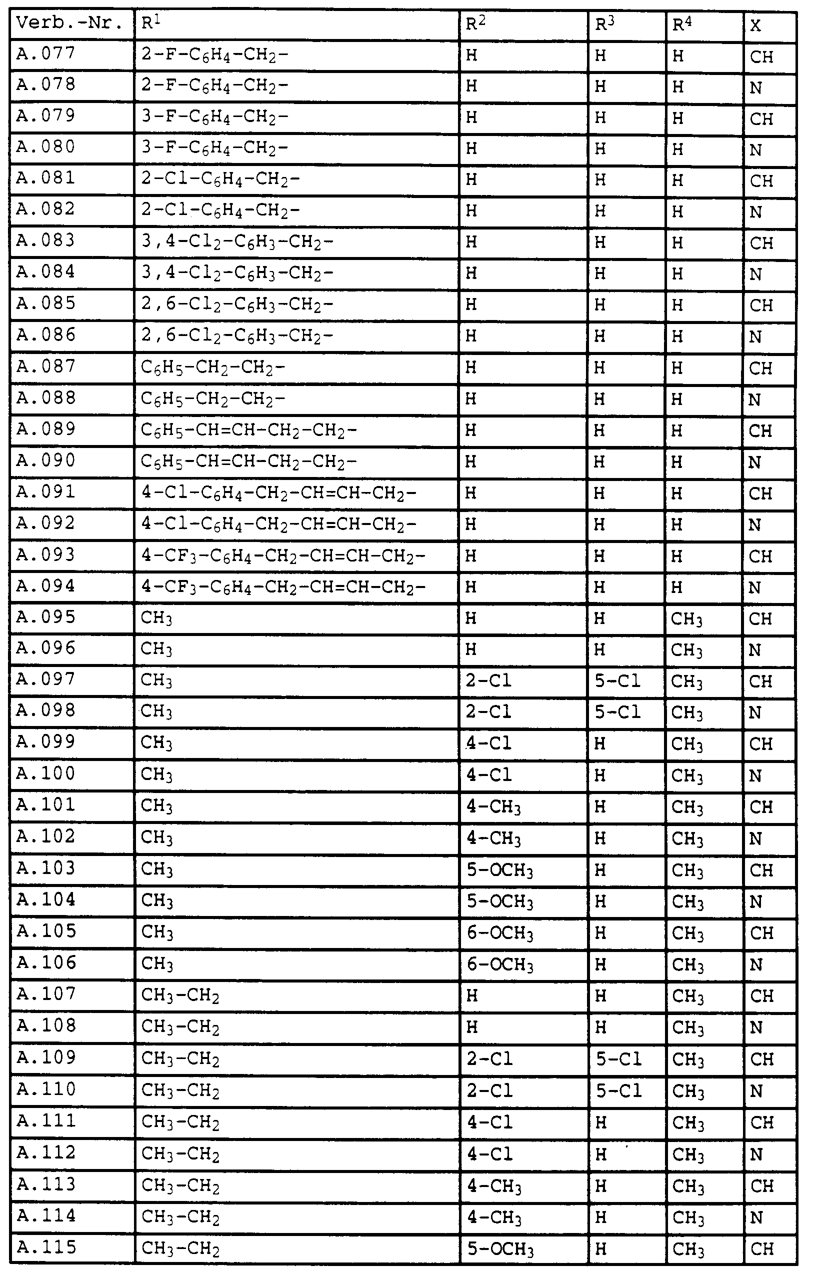

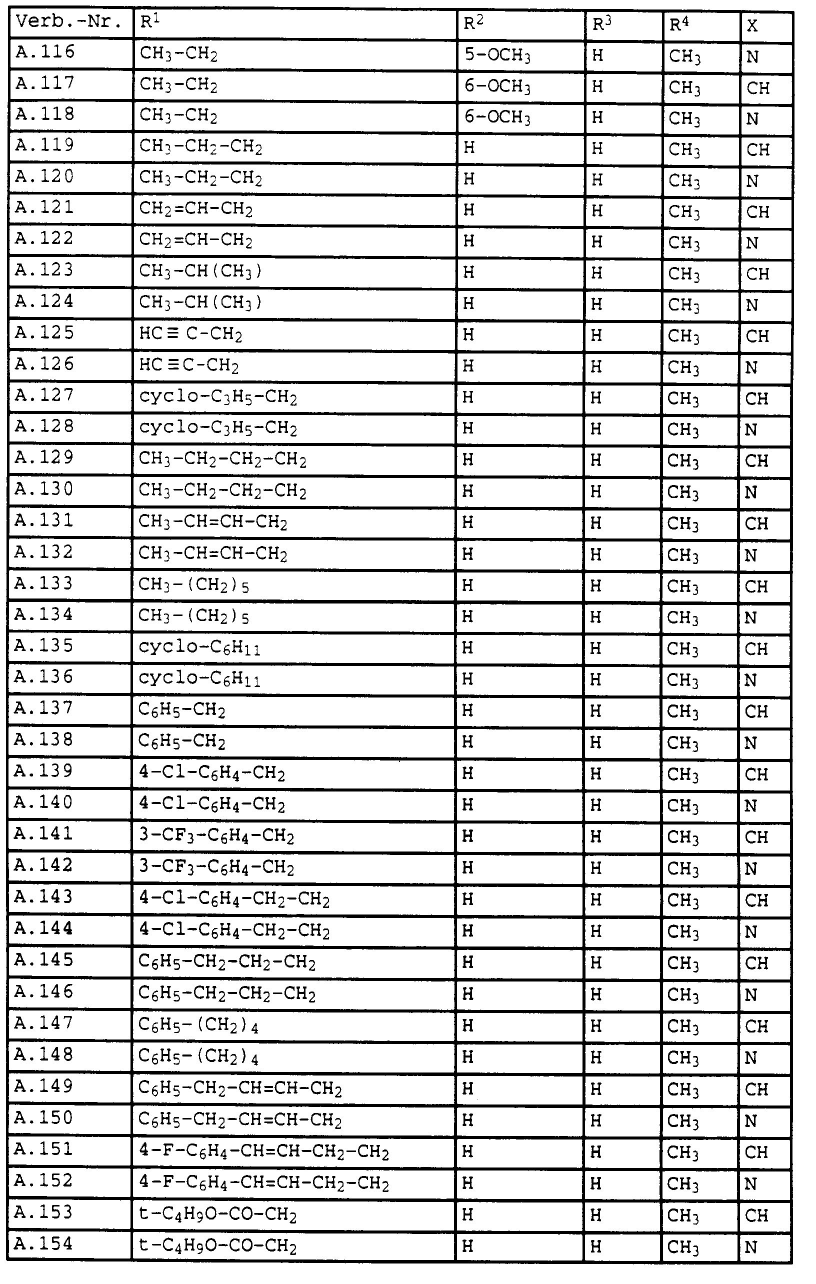

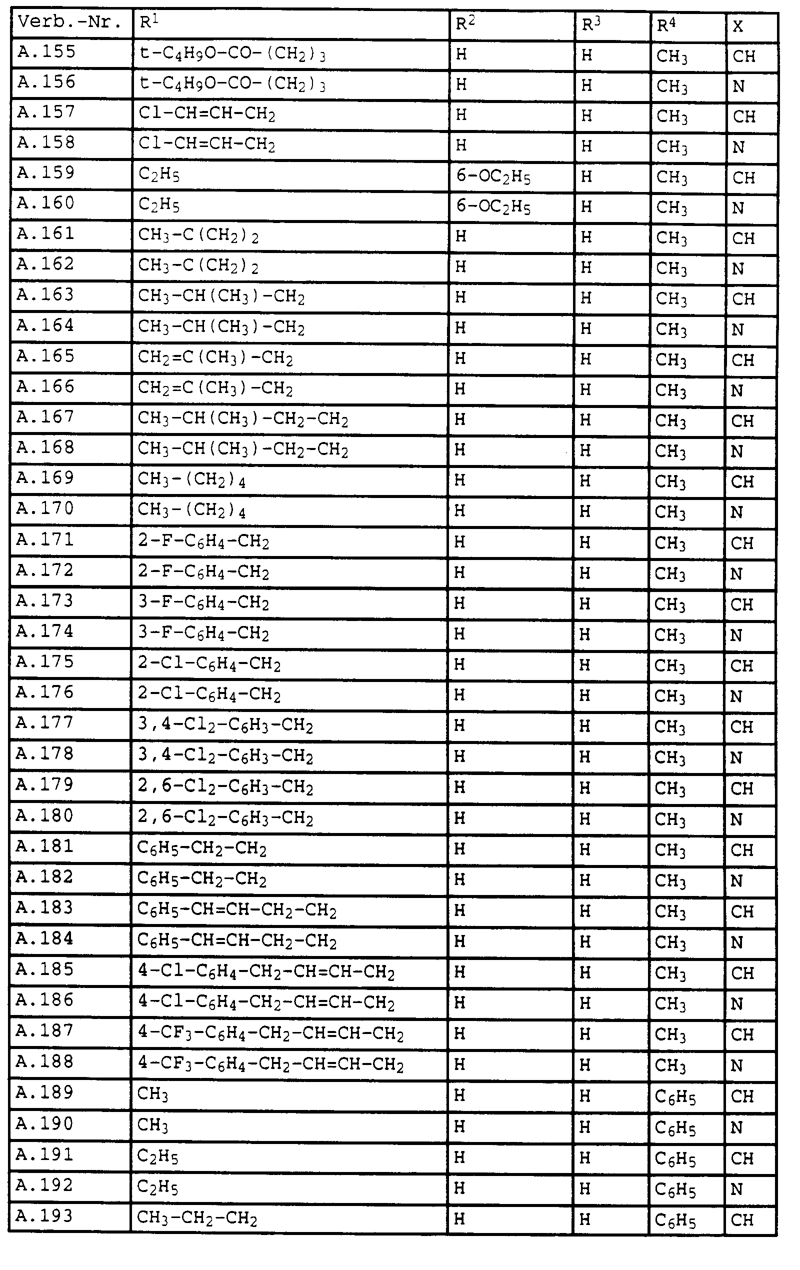

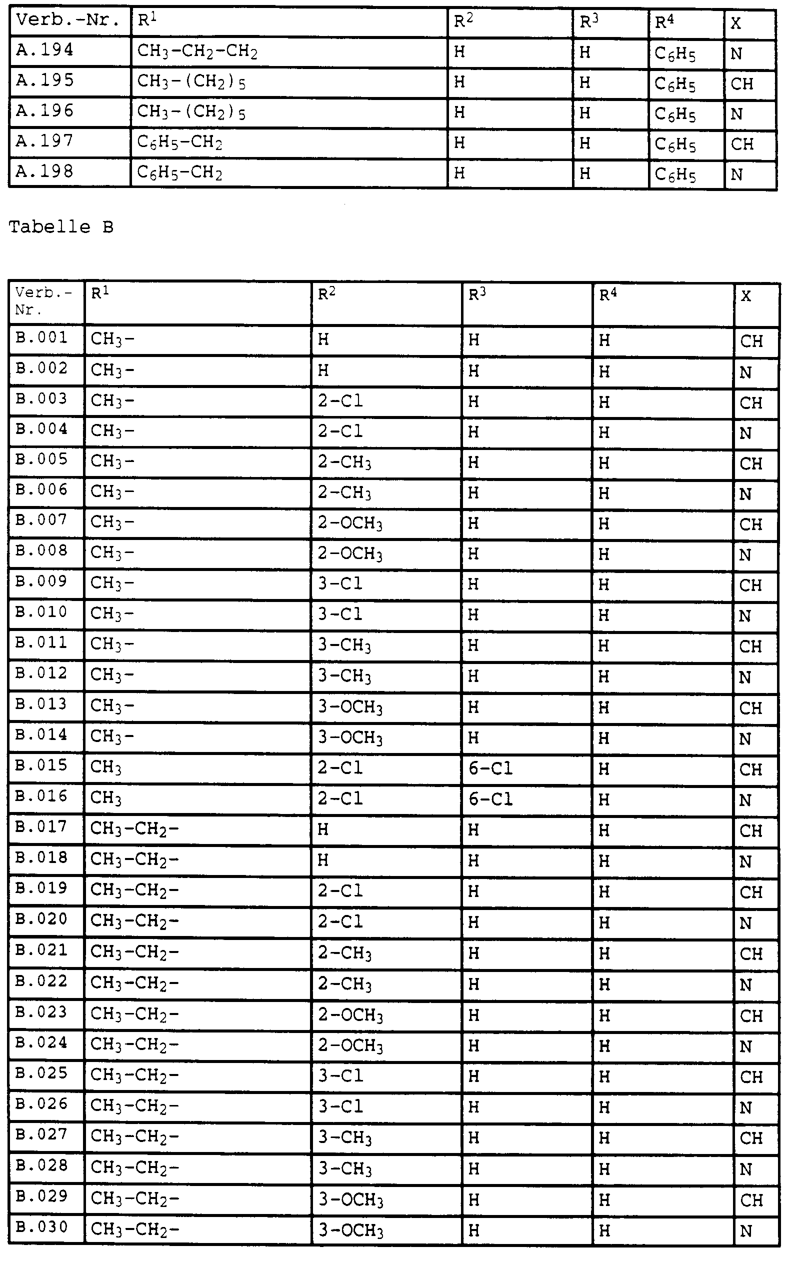

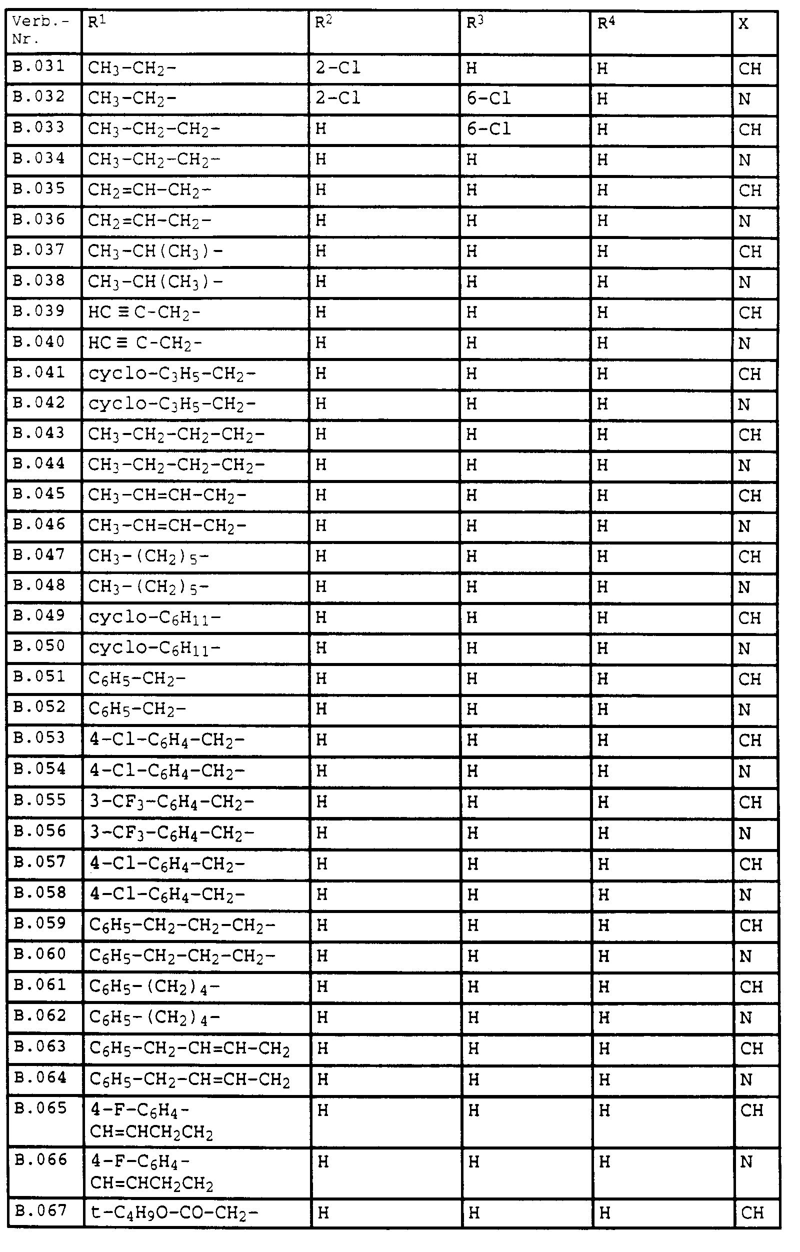

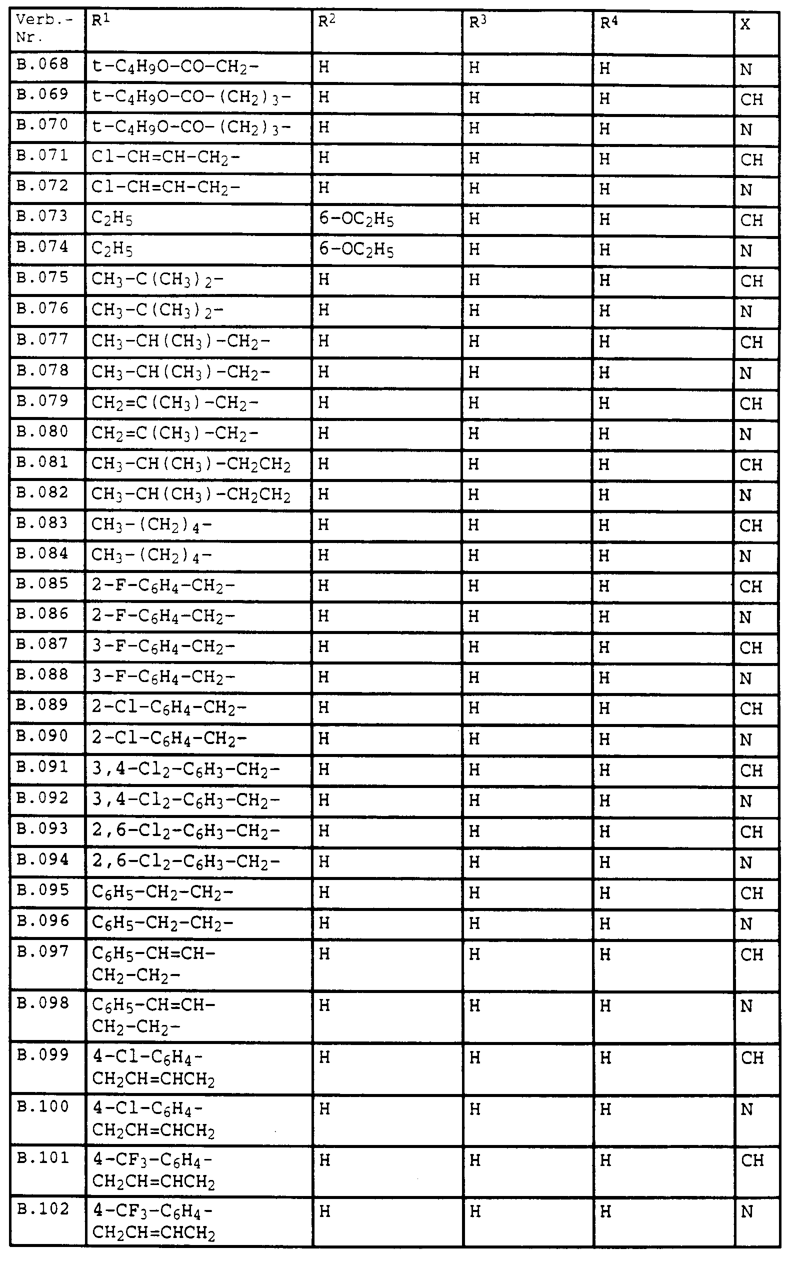

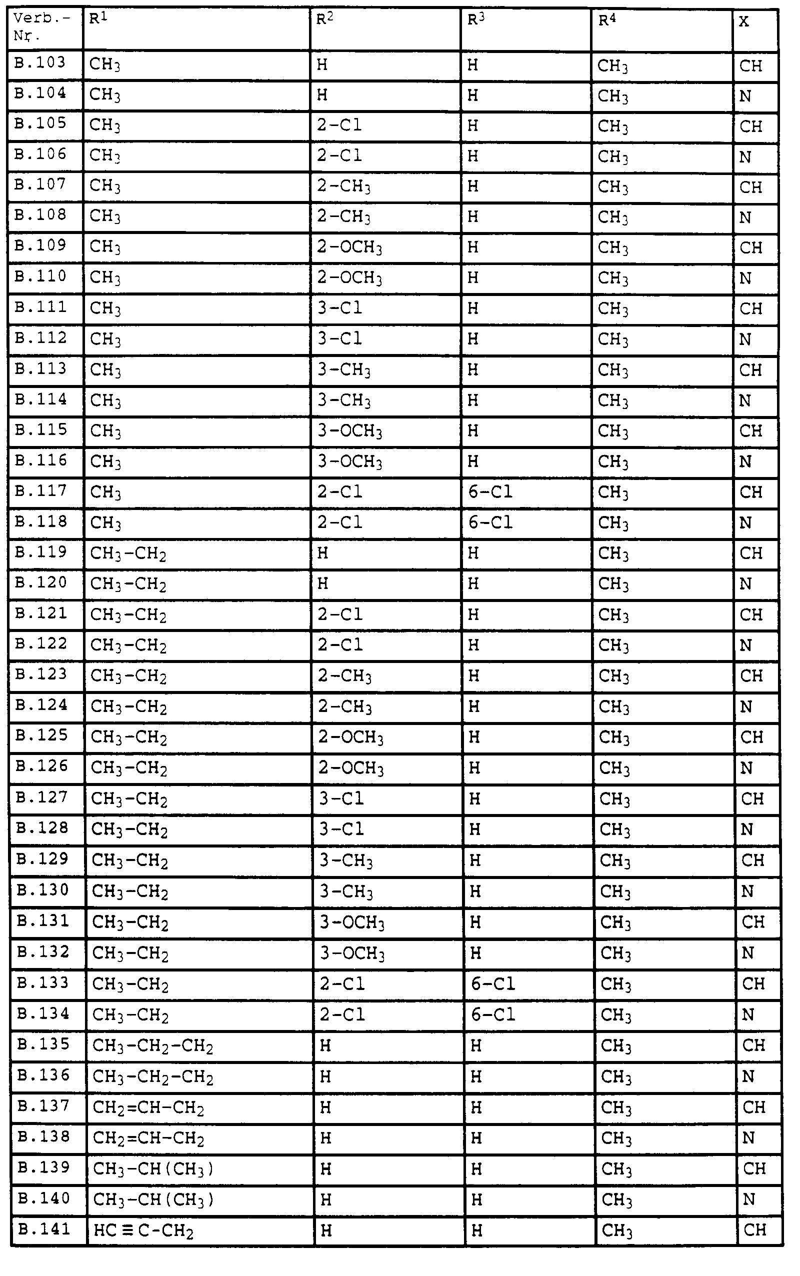

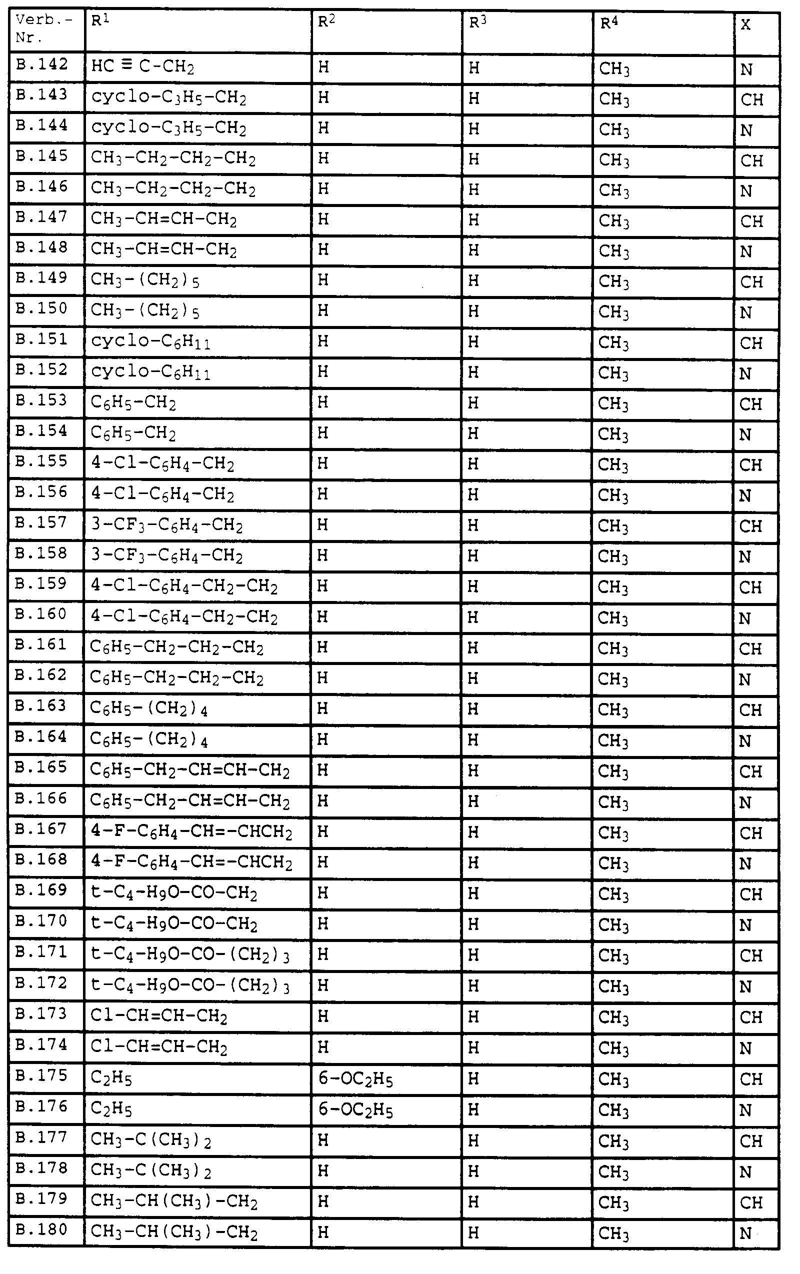

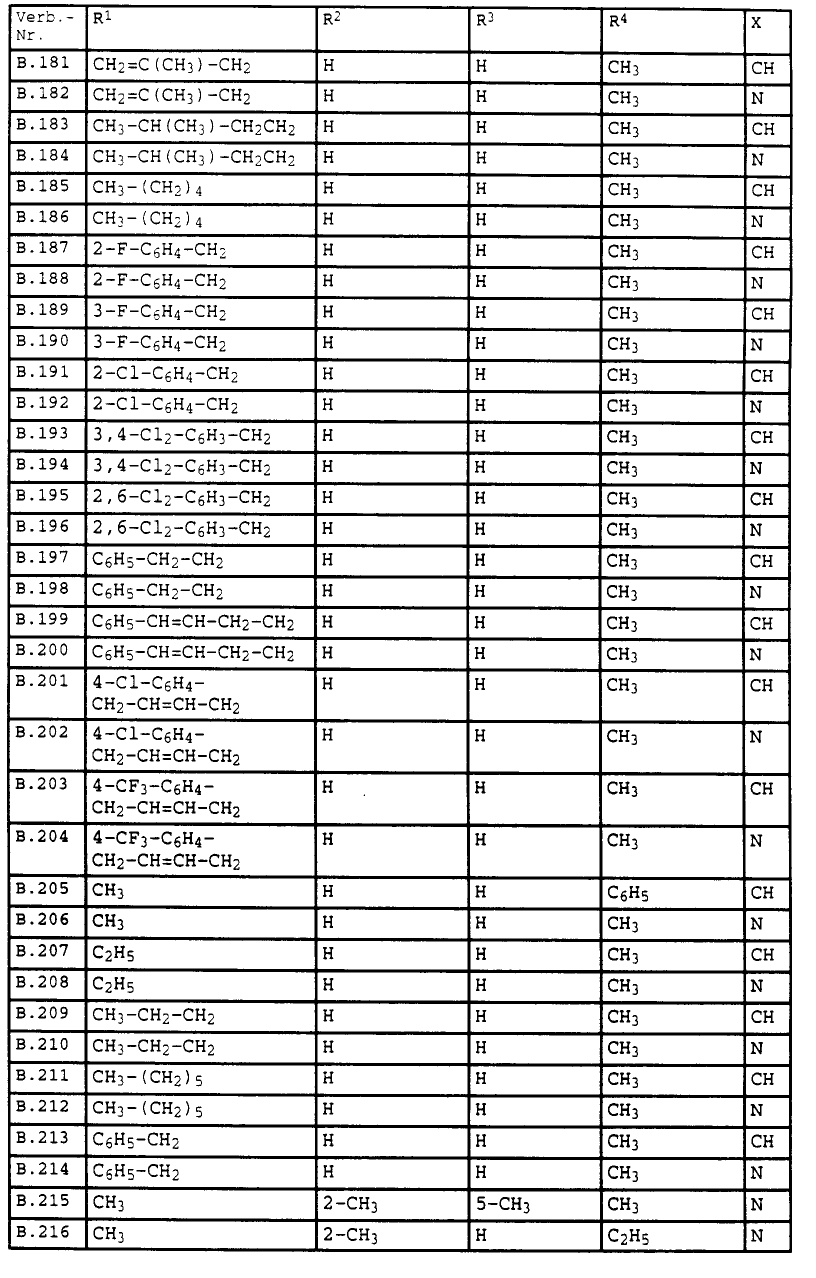

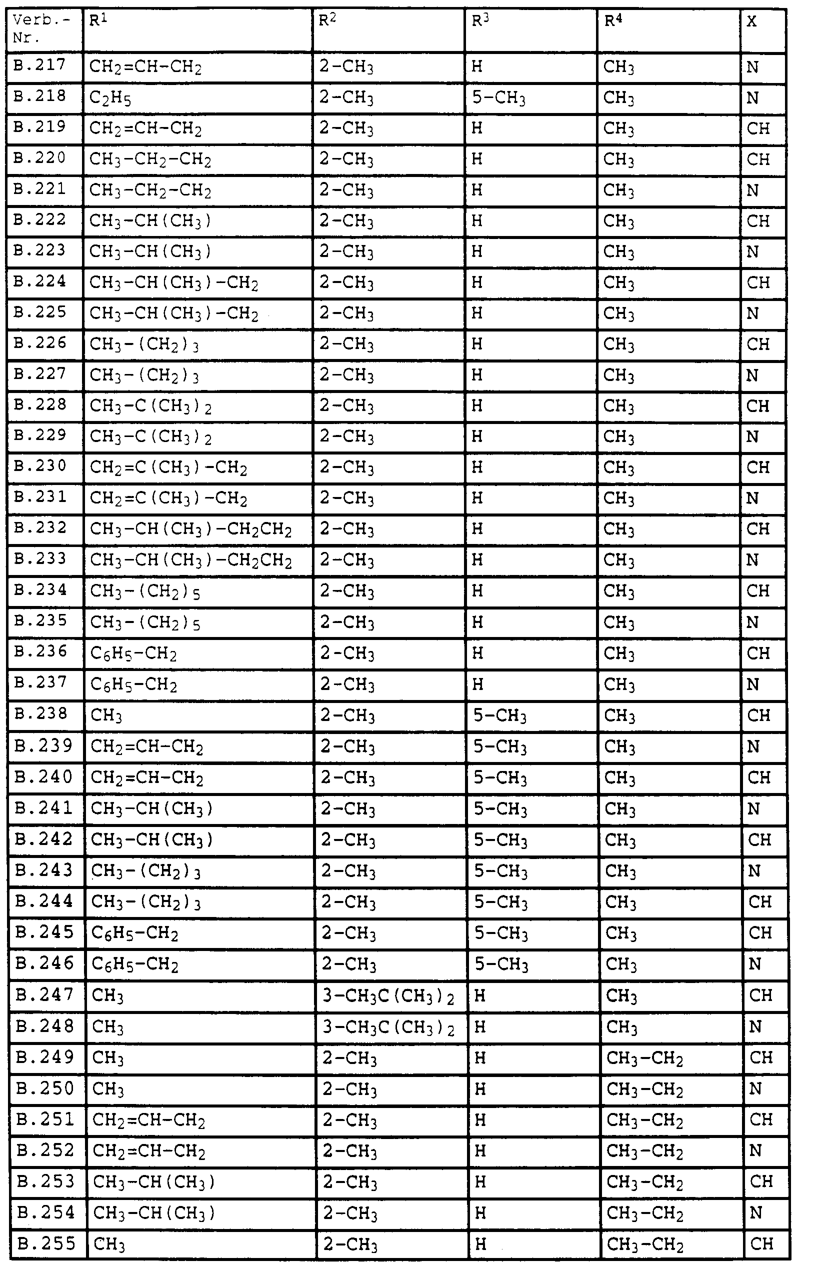

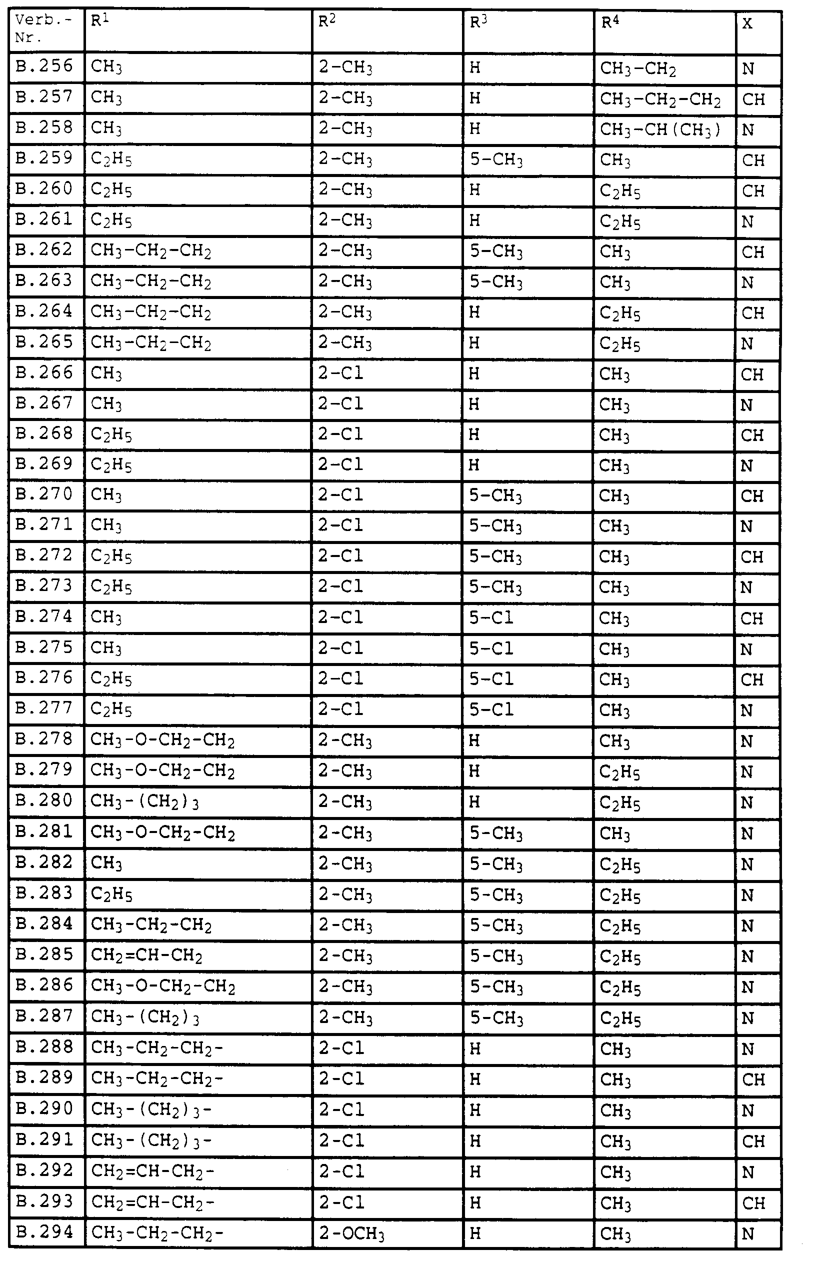

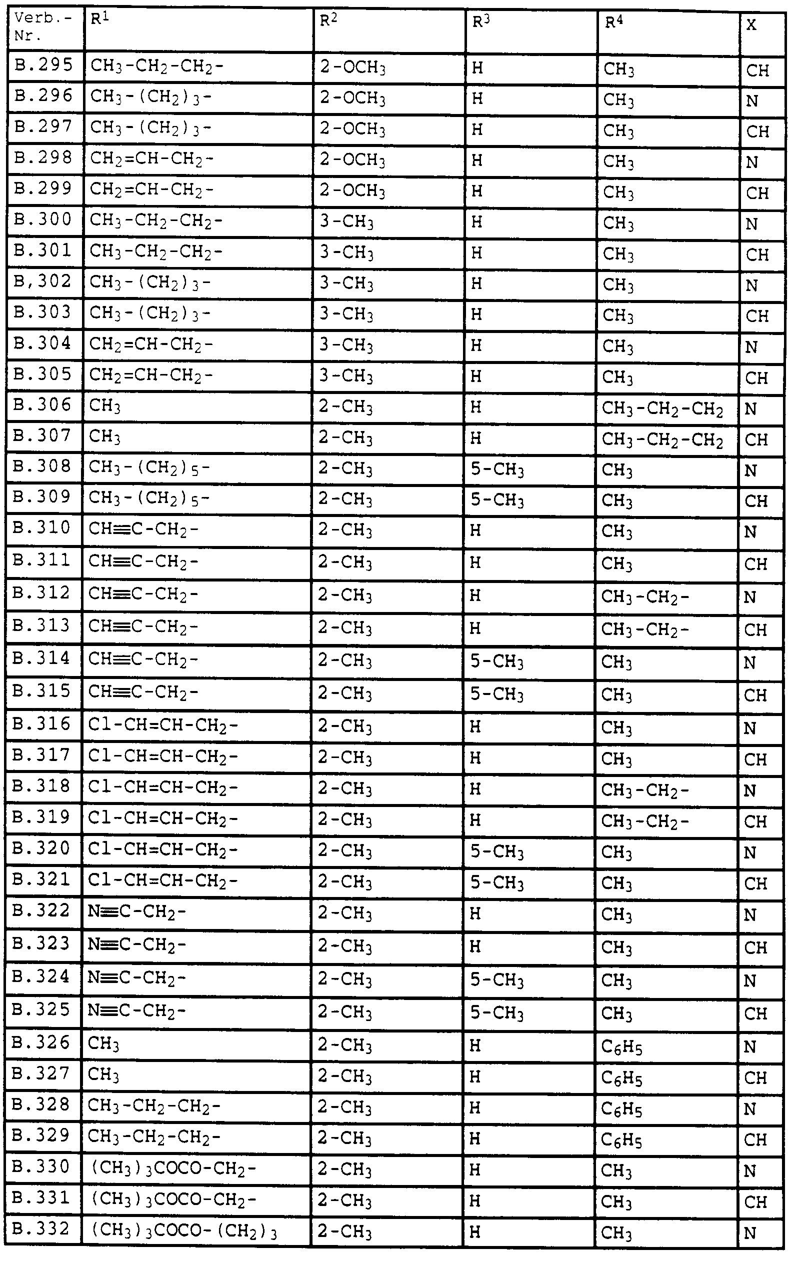

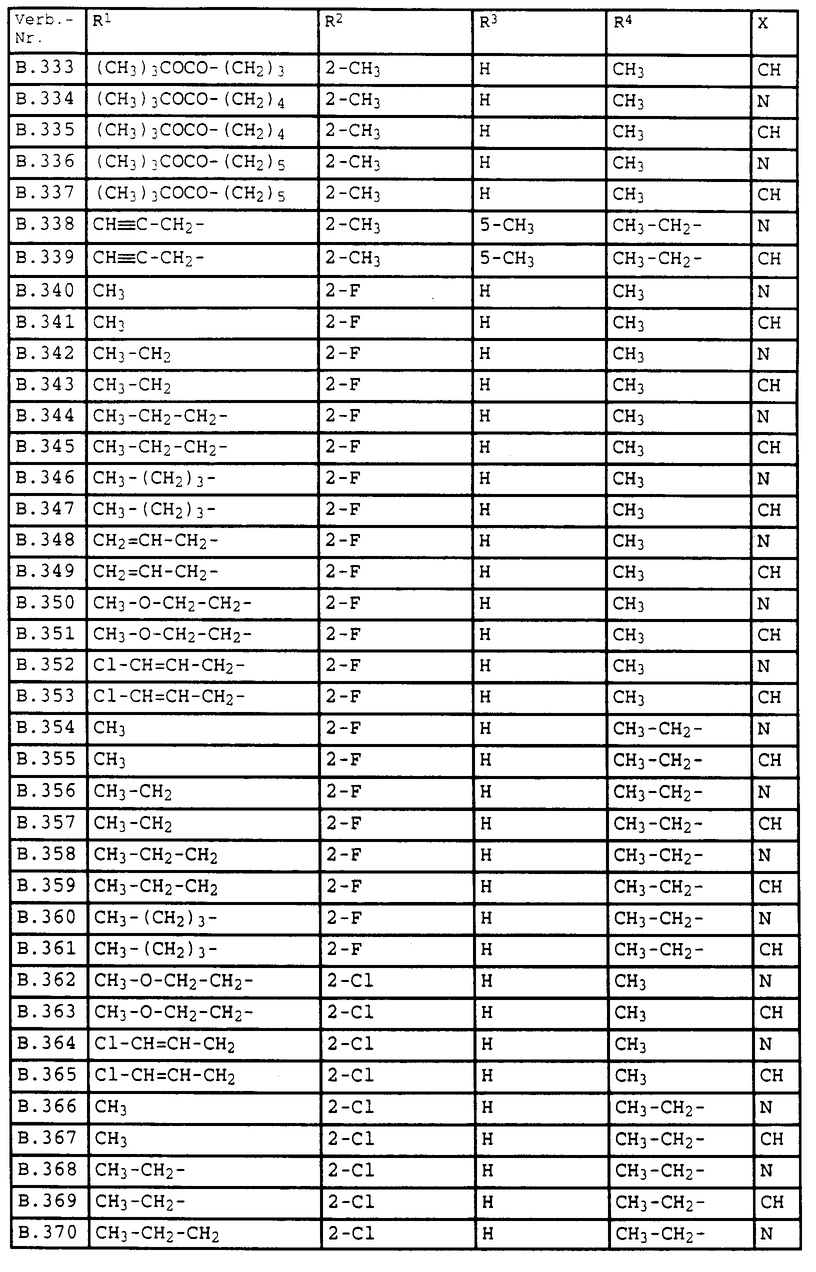

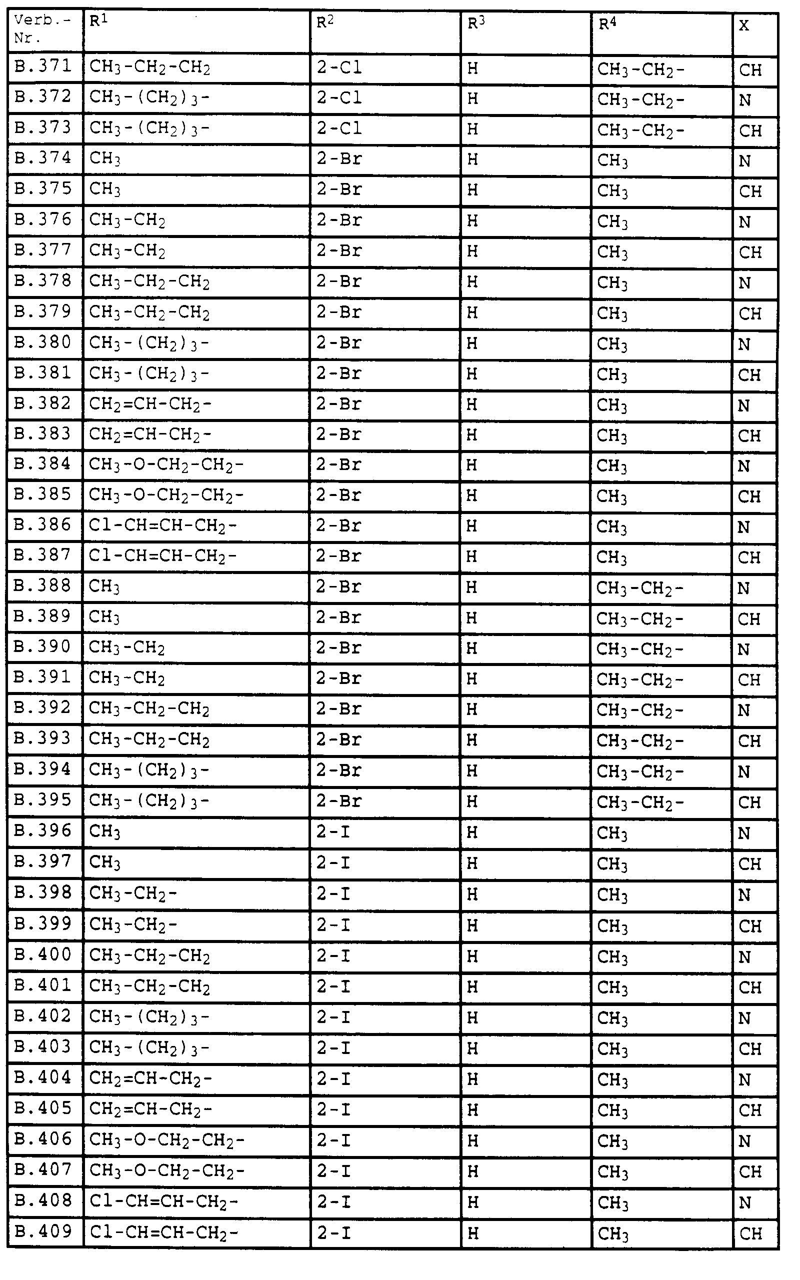

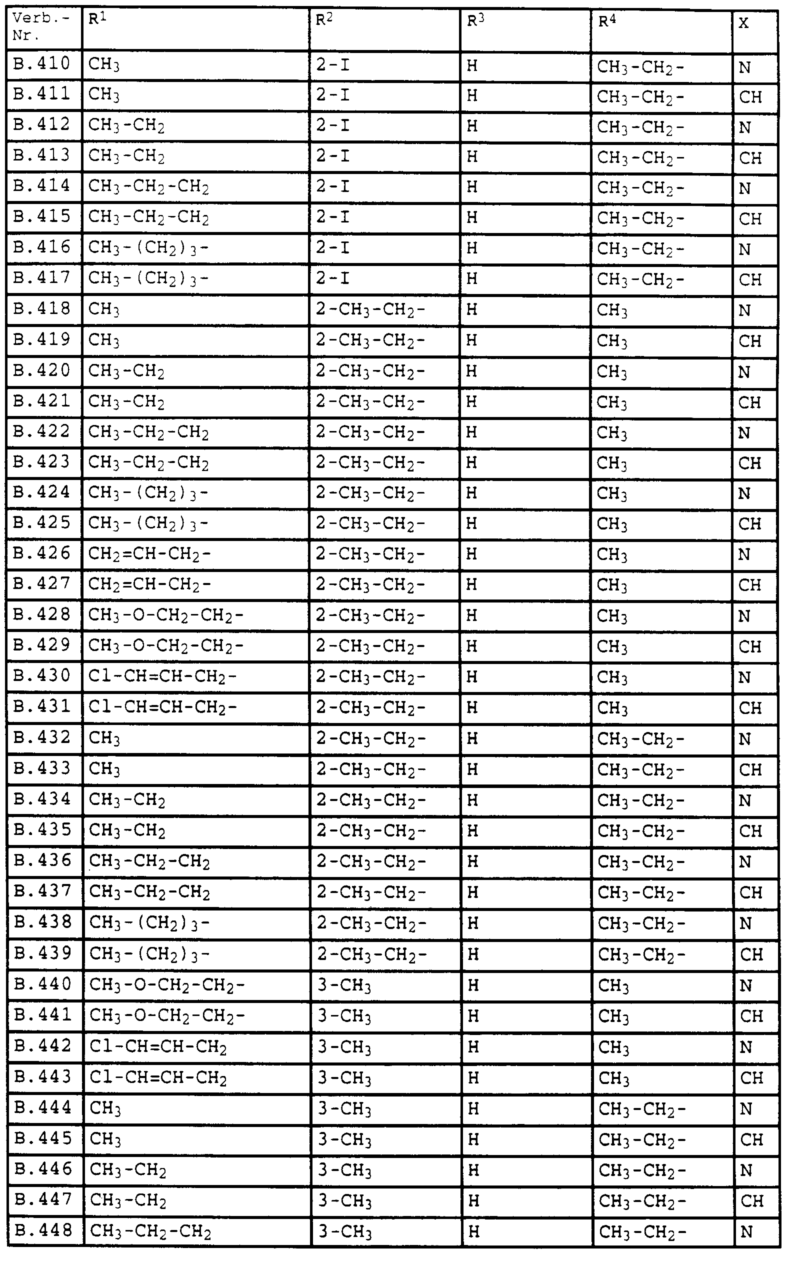

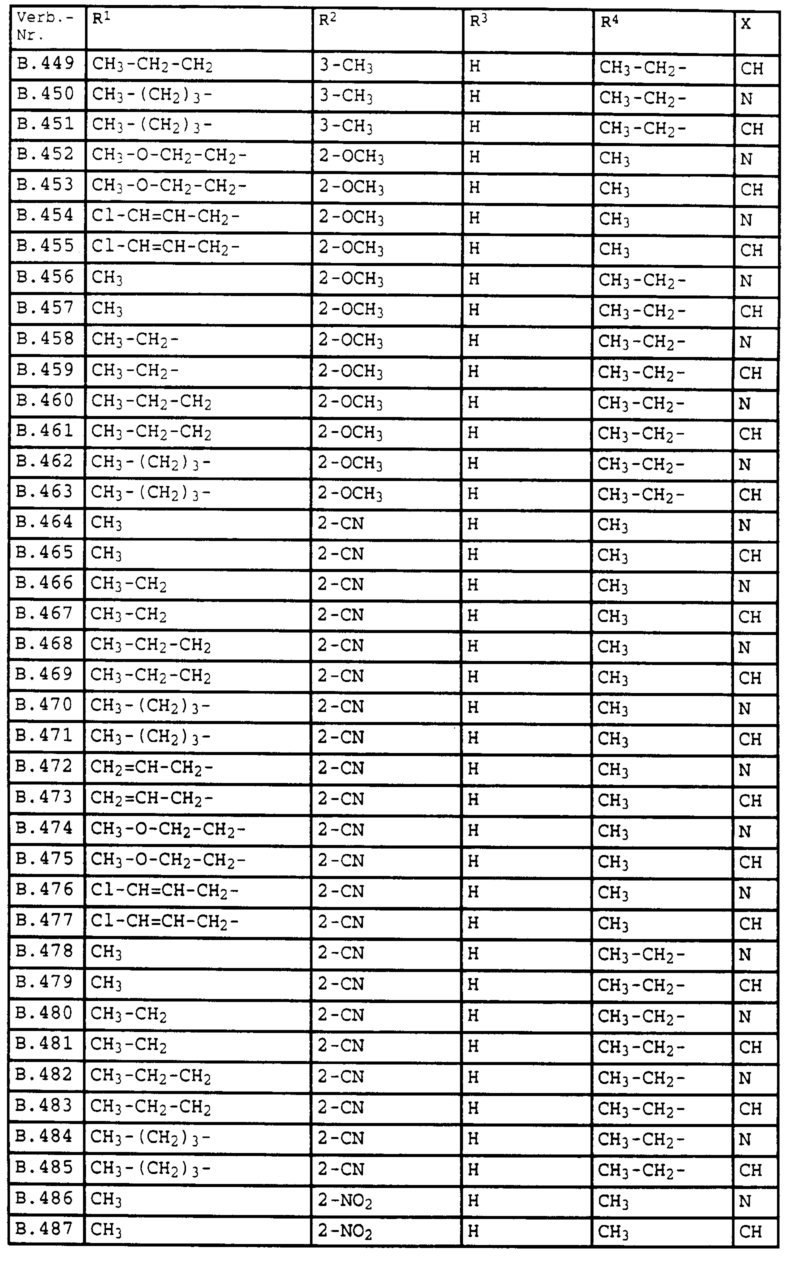

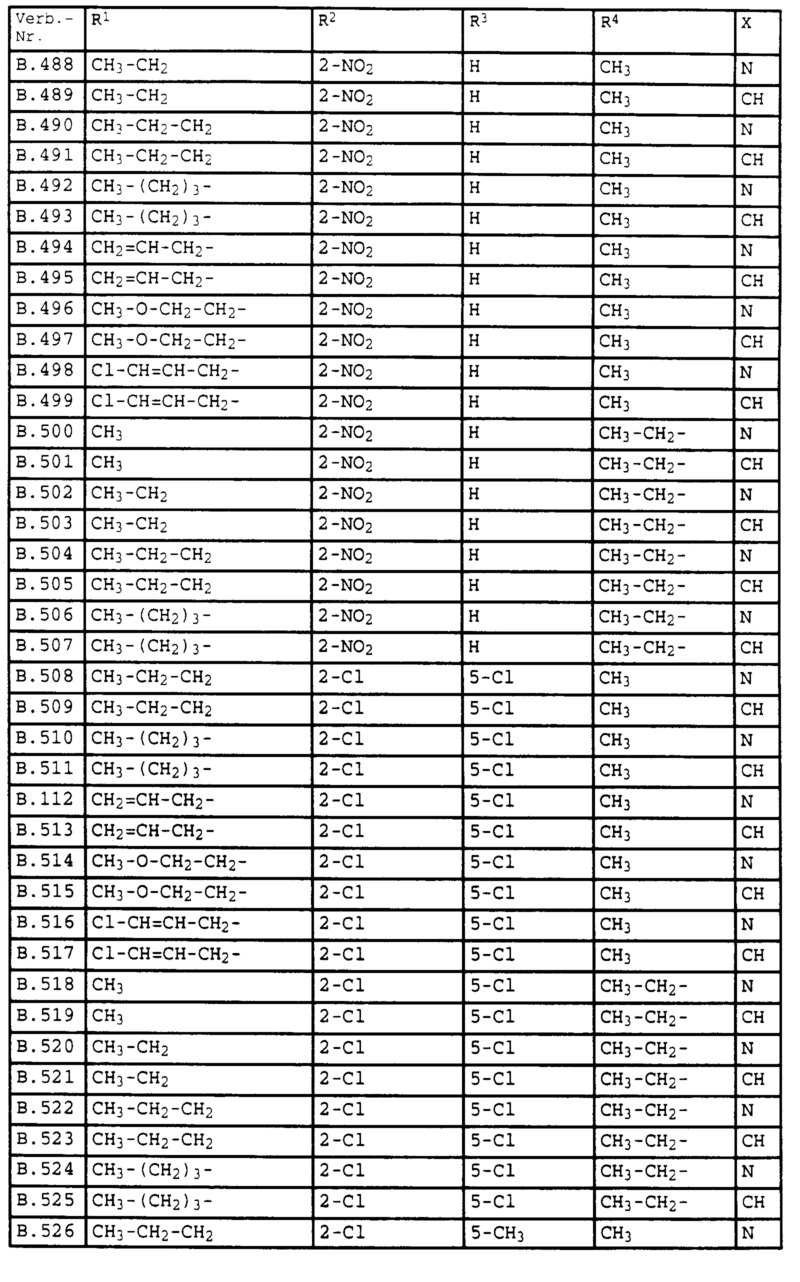

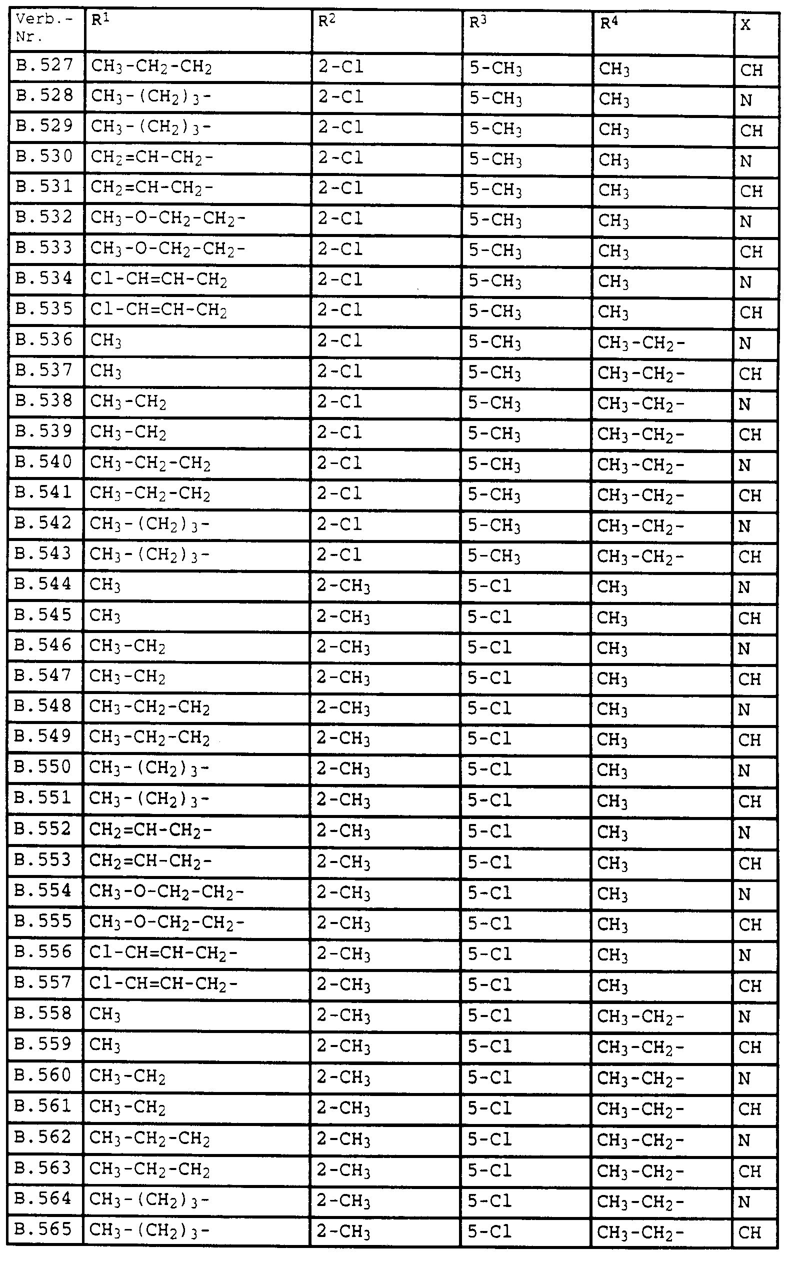

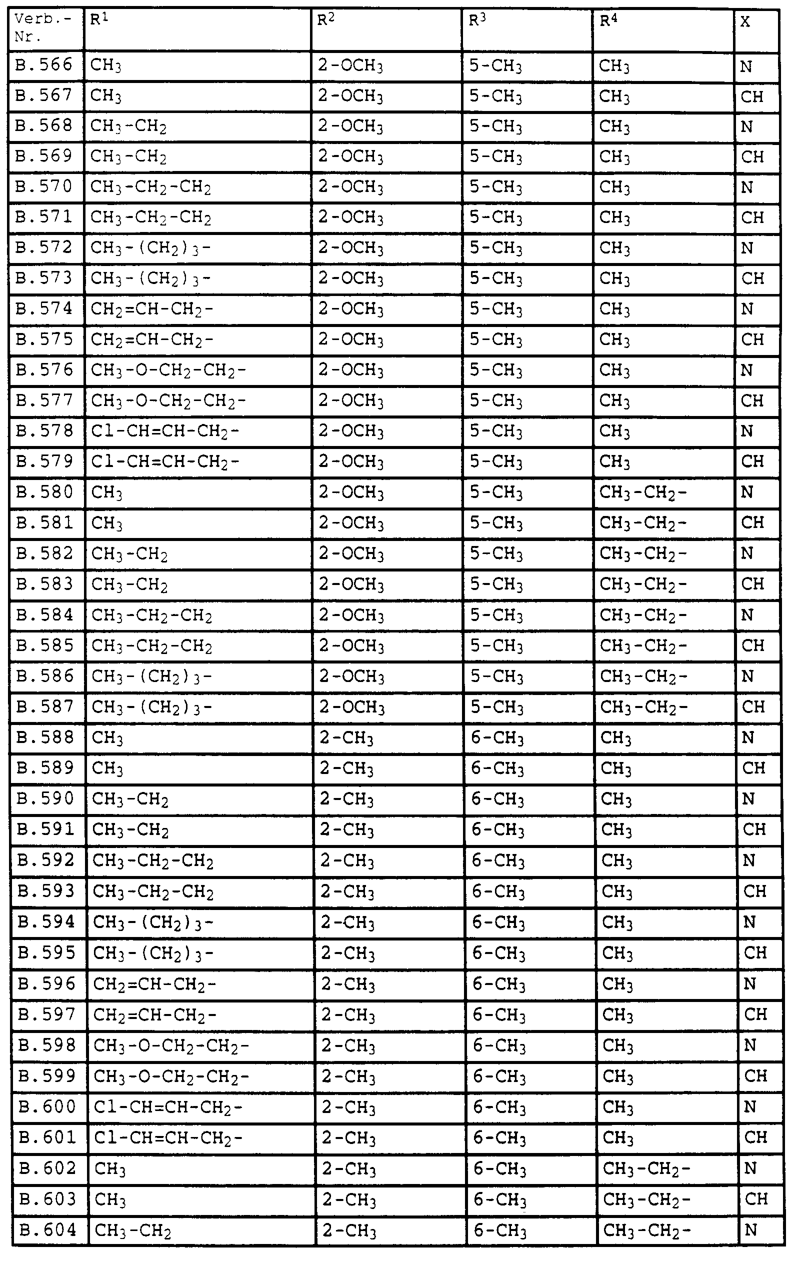

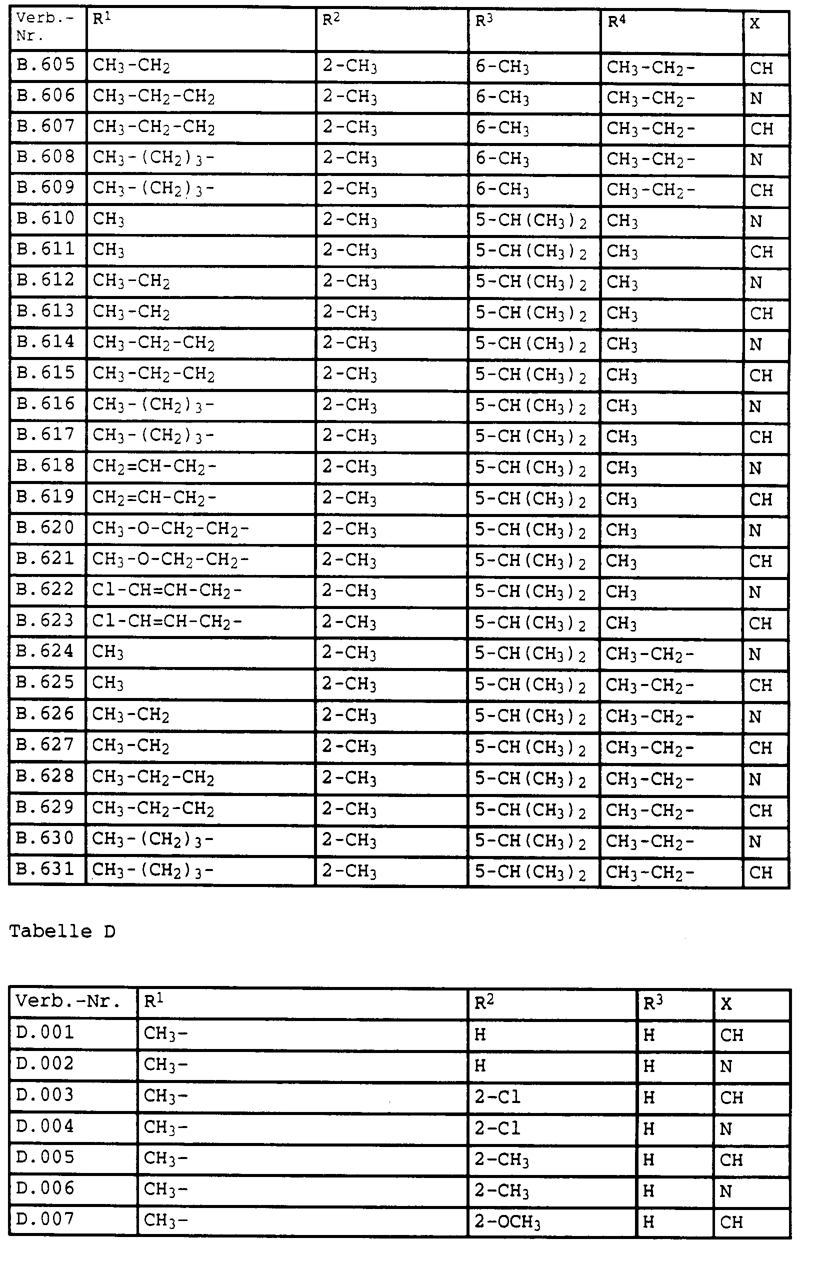

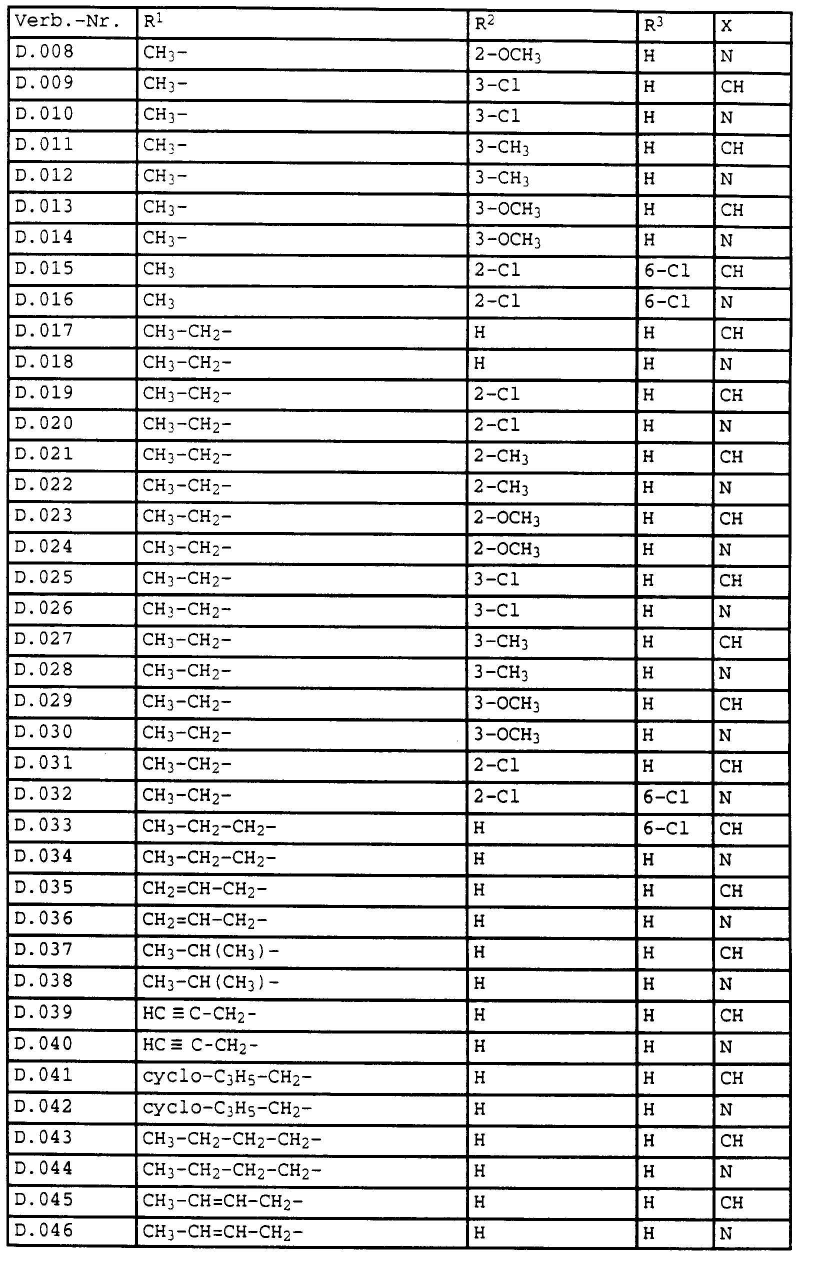

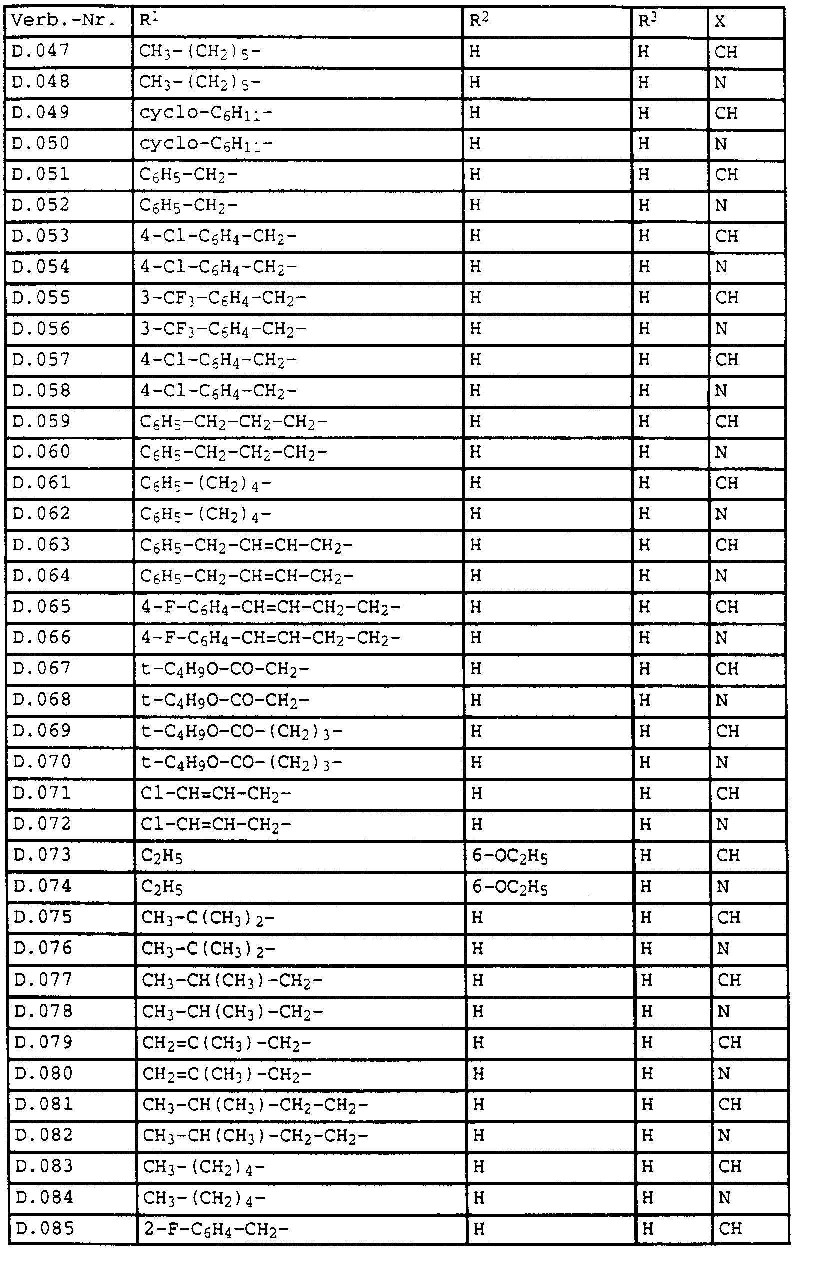

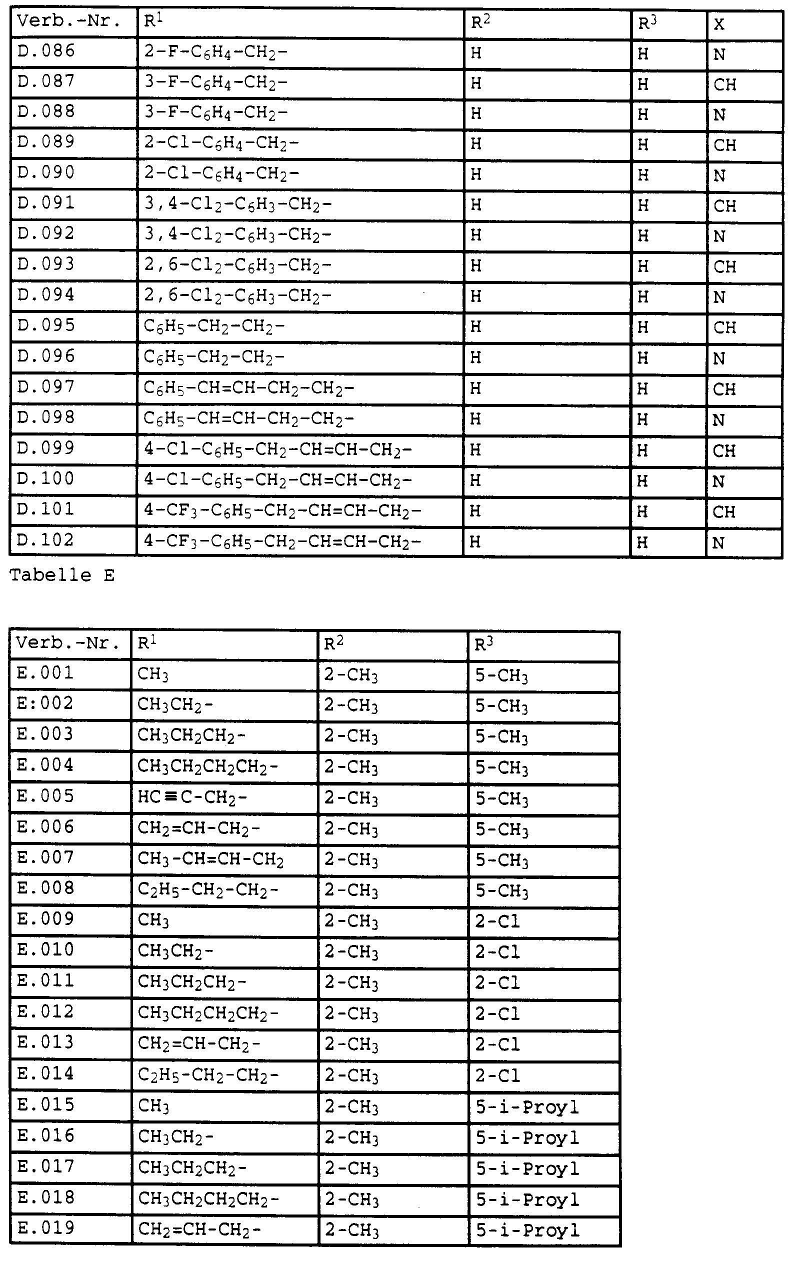

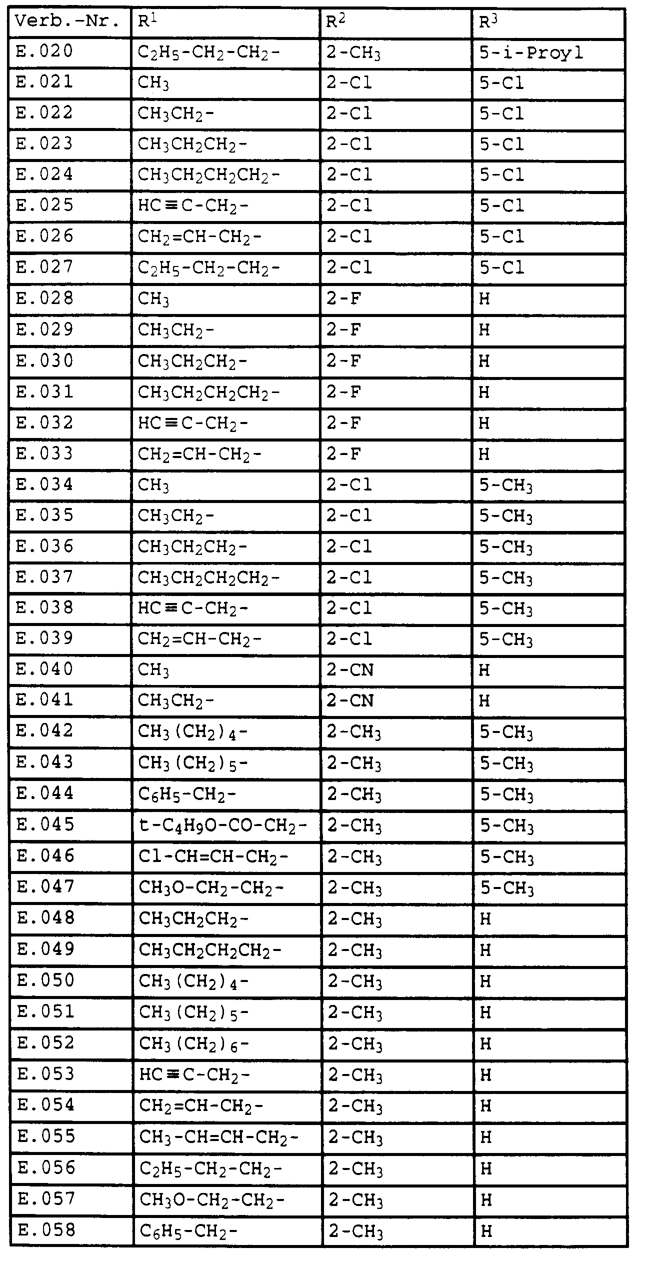

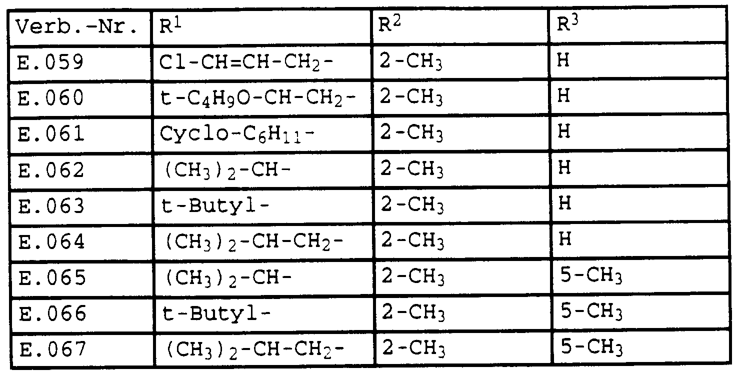

Die Verbindungen der Formel IA sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholita funebrana, Grapholita molesta,Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Phyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthopophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hyoscyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Bemisia tabaci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nephotettix cincticeps, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappahis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Retuculitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Metatetranychus(Phanonychus)ulmi, Ornithodorus moubata, Otobins megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem vis hohem Siedepunkt, wie Kerosin oder Dieselöl, fernen Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser in Betracht.

Wäßrige Anwendungsforman können aus Emulsionskonzentraten, Pasten der netzbaren Pulvern (Spitzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Hetz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Napththalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

### Beispiele für Formulierungen sind:

Granulate, z. B. Umhüllungs-, Imprägnierungs- und Homogengranulate; sie können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

### Anwendungsbeispiele für die Wirkung gegen Schädlinge

Die Wirkung der Verbindungen der allgemeinen Formel IA gegen Schädlinge aus der Klasse der Insekten, Spinntiere und Nematoden ließ sich durch folgende Versuche zeigen:

Die Wirkstoffe wurden
a) als 0,1 %ige Lösung in Aceton oder
b) als 10 %ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindung im Vergleich zu unbehandelten Kontrollversuchen noch eine 80 - %ige Hemmung bzw. Mortalität hervorriefen (Wirkschwelle bzw. Minimal-Konzentration).

### B.1 Aphis fabae (Schwarze Laus), Kontaktwirkung

Stark befallene Buschbohnen (Vicia faba) wurden mit der wäßrigen Wirkstoffaufbereitung behandelt.
Nach 24 h wurde die Mortalitätsrate bestimmt.
In diesem Test zeigten die Verbindungen I.058, I.086 und I.096 Wirkschwellen von 200 bis 1000 ppm.

### B.2 Nephotettix cincticeps (Grüne Reiszikade), Kontaktwirkung

Rundfilter wurden mit der wäßrigen Wirkstoffaufbereitung behandelt und anschließend mit 5 adulten Zikaden belegt.
Nach 24 h wurde die Mortalität beurteilt.
In diesem Test zeigten die Verbindungen I.117, I.307, I.192, I.193, I.195 und I.201 Wirkschwellen von 0,4 bis 0,1 mg.

### B.3 Prodenia litura (Ägypt. Baumwollwurm), Zuchtversuch

Fünf Raupen des Entwicklungsstadiums L3 (10 - 12 mm) wurden auf Standardnährboden (3,1 l Wasser, 80 g Agar, 137 g Bierhefe, 515 g Maismehl, 130 g Weizenkeime sowie übliche Zusatzstoffe und Vitamine (20 g Wessonsalz, 5 g Nipagin, 5 g Sorbin, 10 g Zellulose, 18 g Ascorbinsäure, 1 g Lutavit® blend (Vitamin), 5 ml alkoholische Biotin-Lösung)) aufgebracht, der zuvor mit der wäßrigen Wirkstoffaufbereitung benetzt worden war.
Die Beobachtung erstreckte sich bis zum Schlüpfen der Falter in einem Kontrollversuch ohne Wirkstoff.
In diesem Test zeigten die Verbindungen I.057, I.064, I.068, I.076, I.100, I.108, I.109, I.112, I.119 und I.079 Wirkschwellen von 200 bis 0,1 ppm.

### B.4 Agrotis ypsilon (Erdraupe), Kontaktwirkung

Maisblätter werden für 3 Sekunden in die wäßrige Wirkstoffaufbereitung getaucht und nach dem Abtropfen in eine Petrischale (⌀ 12 cm) auf einen Rundfilter gelegt. Jede Schale wird mit 5 Raupen im 3. und 4. Larvenstadium (ca. 15 mm Länge) belegt.
Nach 24 und 48 Stunden bestimmt man die Wirkung nach % Fraßverhinderung und % Mortalität.
In diesem Test zeigten die Verbindungen I.060, I.070, I.086, I.090, I.096, I.117, I.121, I.129, I.140, I.177, I.307, I.189, I.190, I.191, I.192, I.193, I.195, I.201 und I.213 eine Wirkschwelle von 10 bis 1000 ppm.

### B.5 Sitophilus granaria (Kornkäfer), Kontaktwirkung

Der Boden eines Versuchsgefäßes wurde mit der acetonischen Lösung des Wirkstoffs benetzt und nach dem Abdampfen des Lösungsmittel mit 50 Käfern besetzt.
Nach 4 h wurden die Käfer auf unbehandelte Pappschälchen gesetzt. Diese Schälchen wurden dann in die Versuchsgefäße gestellt.
Nach insgesamt 24 h wurde die Mortalität bestimmt, wobei Käfer, die die Pappschälchen nicht mehr verlassen konnten, als tot bzw. schwer geschädigt galten.
In diesem Test zeigte die Verbindung I.115 eine Wirkschwelle von 1 mg.

### B.6 Musca domestica (Stubenfliege), Kontaktversuch

Der Boden eines Versuchsgefäßes wurde mit der acetonischen Lösung des Wirkstoffs benetzt und nach dem Abdampfen des Lösungsmittel mit 10 Fliegen besetzt.
Nach 4 h wurde die Mortalitätsrate bestimmt.
In diesem Test zeigten die Verbindungen I.064, I.071, I.077, I.078, I.080, I.083, I.085, I.098, I.100, I.103, I.106, I.111, I.115, I.117, I.126, I.127, I.130, I.133, I.309 und I.184 eine Wirkschwelle von 0,01 bis 2 mg.

### B.7 Musca domestica (Stübenfliege), Zuchtversuch

25 ml einer trockenen Futtermischung (1 kg Kleie, 250 g Hefepulver, 35 g Fischmehl) wurde mit dem Wirkstoff und 25 ml einer Milch-Zucker Lösung (1 l Milch, 42 g Zucker) vermischt und anschließend mit 20 Larven des 1. Entwicklungsstadiums besetzt.
Nach dem Schlüpfen der Larven in einem Kontrollexperiment wurde die Mortalität bestimmt.
In diesem Test zeigte die Verbindung I.064 eine Wirkschwelle von 40 ppm.

### B.8 Prodenia litura (Ägypt. Bauwollwurm), Kontaktversuch

Rundfilter (⌀ 9 cm) werden mit 1 cm³ der wäßrigen Wirkstoffaufbereitung behandelt und in eine Kunstoffpetrischale (⌀ 94 mm) gelegt. Anschließend setzt man 5 Prodenia-Raupen L3 ein und verschließt die Petrischale. Die Prüfung erfolgt nach 24 Stunden.
In diesem Test zeigten die Verbindungen I.098, I.100, I.102, I.106, I.111, I.115 und I.184 eine Wirkschwelle von 0,1 bis 1 mg.

### B.9 Prodenia litura (Ägypt. Bauwollwurm), Zuchtversuch

Fünf Raupen des Entwicklungsstadiums L3 (10 - 12 mm) wurden auf Standardnährboden (3,1 l Wasser, 80 g Agar, 137 g Bierhefe, 515 g Maismehl, 130 g Weizenkeime sowie übliche Zusatzstoffe und Vitamine (20 g Wessonsalz, 5 g Nipagin, 5 g Sorbin, 10 g Zellulose, 18 g Ascorbinsäure, 1 g Lutavit®* blend (Vitamin), 5 ml alkoholische Biotin-Lösung)) aufgebracht, der zuvor mit der wäßrigen Wirkstoffaufbereitung benetzt worden war.
Die Beobachtung erstreckte sich bis zum Schlüpfen der Falter in einem Kontrollversuch ohne Wirkstoff.
In diesem Test zeigten die Verbindungen I.128, I.272, I.292, I.293, I.307 und I.310 eine Wirkschwelle von 1 bis 1000 ppm.

### B.10 Plutella maculipennis (Kohlschaben), Kontaktwirkung

Blätter junger Kohlpflanzen wurden mit der wäßrigen Wirkstoffaufbereitung benetzt und anschließend auf einen angefeuchteten Filter gelegt. Die präparierten Blätter wurden anschließend mit jeweils 10 Raupen des 4. Entwicklungsstadiums belegt.
Nach 48 h wurde die Mortalitätsrate bestimmt.
In diesem Test zeigten die Verbindungen I.064, I.065, I.068, I.079, I.081, I.084, I.086, I.088, I.090, I.117 und I.130 eine Wirkschwelle von 200 bis 1000 ppm.

### B.11 Aedes aegypti (Gelbfiebermücke), Zuchtversuch

Kunststoffbecher mit 250 ml Inhalt (⌀ 8 cm) werden mit 200 ml Leitungswasser von 23°C gefüllt und mit 30-40 Aedes-Larven im 3. bis 4. Larvenstadium besetzt. Darauf gibt man die Prüfsubstanz als wäßrige Emulsion und Suspension in das Gefäß und bestimmt nach 24 h die Mortalität in den Gefäßen. Danach züchtet man weiter bis zum Schlüpfen der Mücken. Die Raumtemperatur beträgt 25°C.
In diesem Test zeigte die Verbindung I.128 eine Wirkschwelle von 0,1 ppm.

## Patentansprüche

1. Verfahren zur Bekämpfung von Schädlingen aus der Klasse der Insekten, Spinnentiere und Nematoden, dadurch gekennzeichnet, daß man die Schädlinge und/oder ihren Lebensraum mit einer wirksamen Menge einer Verbindung der allgemeinen Formel IA 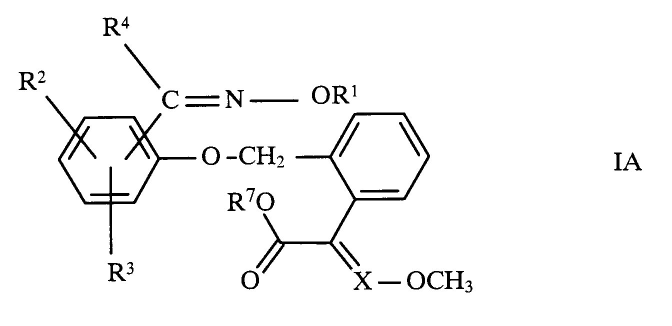 in der
R¹
C₁-C₆-Alkyl, C₃-C₆-Alkenyl, C₃-C₄-Alkinyl, C₁-C₆-Halogenalkyl, C₃-C₆-Halogenalkenyl, C₁-C₄-Alkoxy-C₁-C₆-alkyl, C₃-C₆-Cycloalkyl, C₃-C₆-Cycloalkyl-C₁-C₄-alkyl, Cyan-C₁-C₆-alkyl, C₁-C₆-Alkoxycarbonyl-C₁-C₆-alkyl, Aryl-C₁-C₆-alkyl, Heteroaryl-C₁-C₆-alkyl, Aryl-C₃-C₆-alkenyl oder Aryloxy-C₁-C₆-alkyl bedeutet, wobei der aromatische oder heteroaromatische Ring gegebenenfalls durch einen oder mehrere der folgenden Reste substituiert ist: C₁-C₄-Alkyl, C₁-C₂-halogenalkyl, C₃-C₆-Cycloalkyl, C₁-C₄-Alkoxy, C₁-C₂-Halogenalkoxy, Halogen, Aryl, Aryloxy,
R² und R³
gleich oder verschieden sind und Wasserstoff, C₁-C₄-Alkyl, C₁-C₂-Halogenalkyl, C₁-C₄-Alkoxy, C₁-C₂-Halogenalkoxy, Halogen, Cyano oder Nitro bedeuten,
R⁴
Wasserstoff, C₁-C₆-Alkyl, C₃-C₆-Cycloalkyl, C₁-C₇-Halogenalkyl oder Aryl bedeutet, wobei der aromatische Ring gegebenenfalls durch einen oder mehrere der folgenden Reste substituiert ist: C₁-C₄-Alkyl, C₁-C₂-Halogenalkyl, C₁-C₄-Alkoxy, C₁-C₂-Halogenalkoxy, Halogen, Cyano oder Nitro;
R⁷ C₁-C₄-Alkyl bedeutet, und
X CH oder N bedeutet,
behandelt.

2. Verfahren nach Anspruch 1, dadurch gekennzeichnet, daß man eine Verbindung IA verwendet, in der der Rest -C(R⁴)=NOR¹ in 4-Stellung zur OCH₂-Gruppe steht.

3. Verfahren nach Anspruch 1, dadurch gekennzeichnet, daß man eine Verbindung IA verwendet, in der R⁷ für Methyl steht.

4. Verwendung der Verbindungen IA gemäß Anspruch 1 zur Bekämpfung von Schädlingen aus der Klasse der Insekten, Spinnentiere und Nematoden.

5. Verwendung der Verbindungen IA gemäß Anspruch 1 zur Herstellung eines zur Bekämpfung von Schädlingen aus der Klasse der Insekten, Spinnentiere und Nematoden geeigneten Mittels.

## Claims

1. Method for controlling pests from the class of the insects, arachnids and nematodes, characterized in that the pests and/or their habitat are treated with an effective amount of a compound of the formula IA 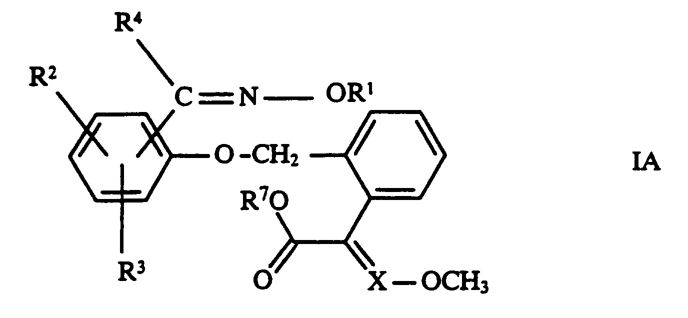 in which
R¹ is
C₁-C₆-alkyl, C₃-C₆-alkenyl, C₃-C₄-alkynyl, C₁-C₆-haloalkyl, C₃-C₆-haloalkenyl, C₁-C₄-alkoxy-C₁-C₆-alkyl, C₃-C₆-cycloalkyl, C₃-C₆-cycloalkyl-C₁-C₄-alkyl, cyano-C₁-C₆-alkyl, C₁-C₆-alkoxycarbonyl-C₁-C₆-alkyl, aryl-C₁-C₆-alkyl, heteroaryl-C₁-C₆-alkyl, aryl-C₃-C₆-alkenyl or aryloxy-C₁-C₆-alkyl, where the aromatic or heteroaromatic ring is unsubstituted or substituted by one or more of the following radicals: C₁-C₄-alkyl, C₁-C₂-haloalkyl, C₃-C₆-cycloalkyl, C₁-C₄-alkoxy, C₁-C₂-haloalkoxy, halogen, aryl, aryloxy,
R² and R³ are
identical or different and are each hydrogen, C₁-C₄-alkyl, C₁-C₂-haloalkyl, C₁-C₄-alkoxy, C₁-C₂-haloalkoxy, halogen, cyano or nitro,
R⁴ is
hydrogen, C₁-C₆-alkyl, C₃-C₆-cycloalkyl, C₁-C₇-haloalkyl or aryl, where the aromatic ring is unsubstituted or substituted by one or more of the following radicals: C₁-C₄-alkyl, C₁-C₂-haloalkyl, C₁-C₄-alkoxy, C₁-C₂-haloalkoxy, halogen, cyano or nitro,
R⁷ is C₁-C₄-alkyl, and
X is CH or N.

2. Method according to Claim 1, characterized in that a compound IA is used in which the radical -C(R⁴)=NOR¹ is in position 4 with regard to the OCH₂ group.

3. Method according to Claim 1, characterized in that a compound IA is used in which R⁷ is methyl.

4. Use of the compounds IA according to Claim 1 for controlling pests from the class of the insects, arachnids and nematodes.

5. Use of the compounds IA according to Claim 1 for preparing a composition suitable for controlling pests from the class of the insects, arachnids and nematodes.

## Revendications

1. Procédé pour combattre les parasites de la classe des insectes, des arachnides et des nématodes, caractérisé par le fait que l'on traite les parasites et/ou leur habitat par une quantité efficace d'un composé de formule générale IA 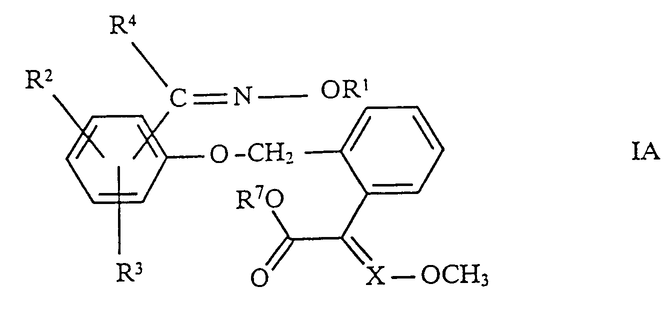 dans laquelle
R¹ représente
un groupe alkyle en C1-C6, alcényle en C3-C6, alcynyle en C3-C4, halogénoalkyle en C1-C6, halogénoalcényle en C3-C6, (alcoxy en C1-C4)alkyle en C1-C6, cycloalkyle en C3-C6, (cycloalkyle en C3-C6)alkyle en C1-C4, cyano-alkyle en C1-C6, (alcoxy en C1-C6)carbonyl-alkyle en C1-C6, aryl-alkyle en C1-C6, hétéroaryl-alkyle en C1-C6, aryl-alcényle en C3-C6 ou aryloxy-alkyle en C1-C6, les novaux aromatiques et hétéroaromatiques pouvant le cas échéant porter un ou plusieurs des substituants suivants : alkyle en C1-C4, halogénoalkyle en C1-C2, cycloalkyle en C3-C6, alcoxy en C1-C4, halogénoalcoxy en C1-C2, halogéno, aryle, aryloxy,
R² et R³, ayant des significations identiques ou différentes, représentent chacun l'hydrogène, un groupe alkyle en C1-C4, halogénoalkyle en C1-C2, alcoxy en C1-C4, halogénoalcoxy en C1-C2, halogéno, cyano ou nitro,
R⁴ représente
l'hydrogène, une groupe alkyle en C1-C6, cycloalkyle en C3-C6, halogénoalkyle en C1-C7 ou aryle, le cycle aromatique pouvant le cas échéant porter un ou plusieurs des substituants suivants : alkyle en C1-C4, halogénoalkyle en C1-C2, alcoxy en C1-C4, halogénoalcoxy en C1-C2, halogéno, cyano ou nitro,
R⁷ représente
un groupe alkyle en C1-C4 et
X représente CH ou N.

2. Procédé selon la revendication 1, caractérisé par le fait que l'on utilise un composé IA pour lequel le groupe -C(R⁴)=NOR¹ est en position 4 par rapport au groupe OCH₂.

3. Procédé selon la revendication 1, caractérisé par le fait que l'on utilise un composé IA pour lequel R⁷ représente un groupe méthyle.

4. Utilisation des composés IA selon la revendication 1 pour la lutte contre les parasites de la classe des insectes, des arachnides et des nématodes.

5. Utilisation des composés IA selon la revendication 1 pour la préparation d'un produit convenant à l'utilisation pour la lutte contre les parasites de la classe des insectes, des arachnides et nématodes.
